# EUROPÄISCHE PATENTSCHRIFT

(11) **EP 3 373 991 B1**
(45) Veröffentlichungstag und Bekanntmachung des Hinweises auf die Patenterteilung: **01.07.2020**
(21) Anmeldenummer: 16801160.9
(22) Anmeldetag: 11.11.2016
(51) Int. Cl.: A61L 27/26, A61L 27/48, A61L 24/00, A61L 24/04

(54) **HOCHSCHLAGZÄHES, TRANSPARENTES PROTHESENMATERIAL MIT NIEDRIGEM REST-MMA GEHALT**
HIGH-IMPACT, TRANSPARENT PROSTHESIS MATERIAL HAVING A LOW RESIDUAL MMA CONTENT
MATÉRIAU DE PROTHÈSE TRANSPARENT À HAUTE RÉSISTANCE AUX CHOCS ET FAIBLE TENEUR RÉSIDUELLE EN MMA

(30) Priorität: 12.11.2015 DE 102015119539
(43) Veröffentlichungstag der Anmeldung: 19.09.2018
(73) Patentinhaber: Kulzer GmbH, 63450 Hanau (DE)
(72) Erfinder: HOHMANN, Alfred, 61389 Schmitten (DE); BUSCH, Susanne, 6820 Frastanz (AT)
(74) Vertreter: Bendele, Tanja
(86) Internationale Anmeldenummer: PCT/EP2016/077420
(87) Internationale Veröffentlichungsnummer: WO 2017/081244

(56) Entgegenhaltungen:
- EP-A2- 1 702 633
- EP-A2- 2 529 762
- WO-A1-2010/051793
- WO-A1-2016/001236
- WO-A1-2016/001242
- DATABASE CA [Online] CHEMICAL ABSTRACTS SERVICE, COLUMBUS, OHIO, US; 12. Mai 1984 (1984-05-12), XP002767200, gefunden im STN-International accession no. 94:175596 Database accession no. 94:175596 & B.M. CULBERTSON ET AL.: "Acryloyloxyalkyl carbamates: synthesis, characterization and some potential uses", ORGANIC COATINGS AND PLASTIC CHEMISTRY (1979), Nr. 40, 1979,
- Anonymous: "CAS # 63225-53-6, 2-[[(Butylamino)carbonyl]oxy]ethyl acrylate, Ebecryl 1029, Ebecryl 1040, Ebecryl CL 1039, Genomer 1122", , 1. Januar 2017 (2017-01-01), XP055344643, Gefunden im Internet: URL:http://www.chemblink.com/products/6322 5-53-6.htm [gefunden am 2017-02-10]

## Beschreibung

Gegenstand der Erfindung ist ein autopolymerisierbares 2-Komponenten-Prothesenbasismaterial, ein Kit enthaltend das Material sowie ein Verfahren zu seiner Herstellung umfassend mindestens eine flüssige Monomerkomponente (A), und mindestens eine pulverförmige Komponente (B), wobei das Prothesenbasismaterial in den Komponenten (A) neben Methylmethacrylat, mindestens ein N-Alkenyl- oder N-Alkyl-substituiertes Acryloyloxy-Carbamat mit einer Molmasse von kleiner gleich 250 g/mol, optional mindestens ein mindestens difunktionelles Urethan(meth)acrylat, mindestens ein di-, tri-, tetra- oder multi-funktionelles Monomer, das kein Urethan(meth)acrylat ist, und optional polymere Partikel mit einer Primärpartikelgröße von kleiner 800 nm, enthält und die pulverförmige Komponente (B) umfasst polymere Partikel mit mindestens drei verschiedenen Partikelgrößenfraktionen und sowohl (A) und (B) enthalten mindestens einen Initiator oder mindestens eine Komponente eines Initiatorsystems für die Autopolymerisation.

Autopolymerisierbare Prothesenbasismaterialien, auch als kalthärtende Prothesenbasismaterialien bezeichnet, haben gegenüber den heißhärtenden Systemen den Vorteil einer einfacheren und schnelleren Verarbeitung. Allerdings enthalten die autopolymerisierbaren Prothesenbasismaterialien in den ersten Tagen nach der Herstellung einen höheren Rest-Methylmethacrylat (MMA) Gehalt. Die Norm für kalthärtende Prothesenbasismaterialien erlaubt bis zu 4.5 Gew.-% an Rest-MMA Gehalt 48 Stunden nach Herstellung. Trotz dieser Beschränkung reagieren immer wieder Patienten allergisch auf ihre Prothese.

Darüber hinaus gibt es bis heute kein kalthärtendes Prothesenbasismaterial mit high Impact-Eigenschaften im Markt, welches die Anforderungen zur erhöhten Schlagzähigkeit nach DIN EN ISO 20795-1 besitzt und darüber hinaus gleichzeitig über eine hohe Transparenz verfügt und damit auch für Aufbiss-Schienen verwendet werden kann.

PalaXpressUltra verfügt über eine erhöhte Bruchzähigkeit, jedoch unterhalb der Normwerte. Dieses Material enthält einen Rest-MMA-Gehalt von 2,9 Gew.-% und verfügt nur über eine mittlere Transparenz von kleiner 85 %. Von Ivoclar wird beispielsweise das SR Ivocap System angeboten, welches über High Impact-Eigenschaften verfügt und einen Rest-MMA-Gehalt von 2.2 Gew.-% Rest-MMA-Gehalt aufweisen soll. Allerdings handelt es sich hierbei um ein Heißpolymerisat. Die entsprechende Norm schreibt einen maximalen Gehalt von 3 Gew.-% Rest-MMA-Gehalt für Heißpolymerisate vor. Nachteilig an diesem System, ist die Notwendigkeit einen hohen Anschaffungspreis für das Ivobase-System auf sich zunehmen, ohne das die Ivocaps nicht sinnvoll verarbeitet werden können. Ferner gibt es kalthärtende Prothesenwerkstoffe mit sehr hoher Transparenz, wie beispielsweise Pala Xpress. Es hat einen Rest-MMA-Gehalt von 3.3 Gew.-%, wobei es jedoch nicht über eine erhöhte Bruchresistenz verfügt.

EP1702633, WO2010051793 und EP2529762 beschreiben autopolymerisierende 2-Komponenten-Prothesenbasismaterialien.

Es besteht ein Bedarf an einem Prothesenwerkstoff, welcher kurzzeitige Belastungen, wie die vorgenannten kurzzeitigen, hohen mechanischen Belastungen, ohne Materialschädigung toleriert und gleichzeitig über die Vorzüge eines autopolymerisierbaren Kunststoffes verfügt. Diese Prothesenwerkstoffe werden als kalthärtende High Impact-Werkstoffe bezeichnet. Die Anforderungen an diese Werkstoffe sind in der DIN ISO 20795-1 beschrieben. High-Impact-Materialien sind schon seit einigen Jahren auf dem Markt, diese sind allerdings ausschließlich der Gruppe der heisshärtenden Prothesenwerkstoffe zuzuordnen.

Aufgabe der Erfindung war die Bereitstellung einer Materials, insbesondere eines im medizinischen Bereich geeigneten Materials, bevorzugt eines Prothesenbasismaterials zur Kaltpolymerisation, das die Vorgaben der Norm DIN ISO 20795-1 hinsichtlich Bruchzähigkeit erfüllt und vorzugsweise übertrifft. Zudem soll das Material eine deutlich verbesserte Transparenz aufweisen. Eine weitere Aufgabe bestand darin, einen sehr niedrigen Rest-MMA Gehalt in dem polymerisierten Material für ein Kaltpolymerisat zu erhalten. Ferner bestand die Aufgabe, dass das Material ohne aufwendige Zusatzgeräte herstellbar sein soll, insbesondere soll auch eine Verarbeitung ohne spezielle Zusatzgeräte möglich sein. Das Material soll mit typischen zahntechnischen Techniken und Geräten zu verarbeiten sein. Eine weitere Aufgabe bestand darin, einen hoch-bruchzähen Werkstoff, insbesondere einen Prothesenwerkstoff mit sehr hoher Transparenz bereitzustellen.

Es wurde überraschend gefunden, dass mit dem erfindungsgemässen 2-Komponenten Prothesenbasismaterial erhältlich durch Vermischen und Polymerisieren unter den Bedingungen einer Kaltpolymerisation bzw. Autopolymerisation der Komponente (A) und der Komponente (B) ein Material erhältlich ist, welches eine sehr hohe Transparenz, hohe Bruchzähigkeit und einen niedrigen Rest-MMA-Gehalt (Rest-Methylmethacrylat-Gehalt), in einem einzigen Material vereint und dennoch einfach entsprechend einem kalthärtenden Kunststoff verarbeitet werden kann.

Gegenstand der Erfindung ist das auspolymerisierte Prothesenbasismaterial mit einem Rest-MMA-Gehalt, nach Norm ISO 20795-1:2013 von maximal 2.2 Gew.-%, einer Bruchzähigkeit ausgedrückt als Höchstfaktor der Beanspruchungsintensität von größer/gleich 2 MPa/m² und einer Gesamtbrucharbeit von größer gleich 1200 J/m², welches darüber hinaus eine sehr hohe Transparenz von größer 90 % besitzt. Aufgrund der hohen Transparenz ist das Material z.B. auch für Aufbissschienen und Bohrschablonen für Implantatarbeiten geeignet.

Gegenstand der Erfindung ist ein autopolymerisierbares 2-Komponenten-Prothesenbasismaterial, insbesondere erhältlich durch Mischen mindestens einer flüssigen Monomerkomponente (A) und Polymerisation unter den Bedingungen der Autopolymerisation, und mindestens einer pulverförmigen Komponente (B), wobei das Prothesenbasismaterial umfasst mindestens eine flüssige Monomerkomponente (A), und mindestens eine pulverförmige Komponente (B), wobei die Komponente (A) umfasst
(i) mindestens einen Methylmethacrylat sowie optional mindestens ein (2-Alkyl)-Acrylsäureester, das nicht Methylmethacrylat ist,
(ii) mindestens ein N-Alkyl- oder N-Alkenyl-substituiertes Acryloyloxy-Carbamat mit einer Molmasse von kleiner gleich 250 g/mol,
(iii) optional mindestens ein mindestens difunktionelles Urethan(meth)acrylat,
(iv) mindestens ein di-, tri-, tetra- oder multi-funktionelles Monomer, das kein Urethan(meth)-acrylat ist,
(v) optional polymere Partikel mit einer Primärpartikelgröße von kleiner 800 nm, insbesondere Kern-Schale Partikel, insbesondere mit einer Primärpartikelgröße von 500 nm bis 10 nm,
(vi) mindestens einen Initiator oder mindestens eine Komponente eines Initiatorsystems für die Autopolymerisation, und Komponente (B) umfasst
   (i) mindestens eine pulverförmige Komponente polymerer Partikel, die mindestens drei verschiedene Fraktionen von Partikelgrößen polymerer Partikel umfasst, insbesondere ist die mittlere Partikelgröße jeder Faktion der mindestens drei Fraktionen von Partikelgrößen um mindestens 5 Mikrometer von der mittleren Partikelgröße der beiden anderen Fraktionen beabstandet, und vorzugsweise liegen die mittleren Partikelgrößen aller Fraktionen im Bereich von 10 bis 120 Mikrometer, insbesondere von 30 bis 65 Mikrometer, bevorzugt sind die drei Fraktionen ausgewählt
      1) aus polymeren Partikeln einer mittleren Partikelgröße von
         a) von 25 bis kleiner 40 µm, insbesondere 30 bis kleiner 40 µm, bevorzugt 35 µm mit plus/minus 2,5 µm
         b) von 40 bis kleiner 55 µm, insbesondere von 40 bis 50 µm, bevorzugt 45 µm mit plus/minus 2.5 µm
         c) von 55 bis 100 µm, insbesondere 55 bis 80 µm, bevorzugt 55 bis 65 µm,) weiter bevorzugt 60 µm mit plus/minus 2,5 µm, oder bevorzugt
      2) aus polymeren Partikeln einer mittleren Partikelgröße von
         a) von 35 µm mit plus/minus 2,5 µm
         b) von 45 µm mit plus/minus 2,5 µm
         c) von 60 µm mit plus/minus 2,5 µm, wobei das Gewichtsverhältnis von a) zu b) zu c) von 12 bis 18 zu 1 zu 1 bis 5 beträgt, vorzugsweise von 14 bis 17 zu 1 zu 2 bis 4, besonders bevorzugt von 15 bis 16 zu 1 zu 3 mit einer Schwankungsbreite von +/- 0,5, sowie
   (ii) mindestens einen Initiator oder mindestens eine Komponente eines Initiatorsystems für die Autopolymerisation.

Um erfindungsgemäss den Restmonomergehalt an MMA zu reduzieren, ist zum einen ein Austausch des MMAs durch andere Komponenten möglich und optional auch eine Erhöhung des Polymerisationsgrades durch eine geeignete Temperaturführung. Die Temperaturentwicklung während der Polymerisation wird maßgeblich durch das Initiatorsystem, das Monomersystem und die eingesetzten Polymer-Perlen beeinflusst. Diese Komponenten müssen in geeigneter Weise auf einander abgestimmt werden. Eine starke Temperaturentwicklung im Rahmen der Polymerisation führt zwar in der Regel zu einem höheren Polymerisationsgrad und einer hohen Biegefestigkeit, jedoch auf Kosten der Passgenauigkeit, da der Schrumpf sehr hoch ist und auf Kosten der Bruchzähigkeit. Darüber hinaus können Luft-Blasen im Produkt auftreten. Ist die Temperatur andererseits während der Polymerisation zu niedrig, kann dies die Transparenz und Robustheit negativ beeinflussen. Ferner reichen dann die üblichen Polymerisationszeiten nicht aus, um die Endhärte des Materials und den entsprechend der oben genannten Norm verlangten Rest-MMA-Gehalt zu erreichen. Die mechanischen Eigenschaften werden zudem von der Auswahl der richtigen Polymer-Perlen beeinflusst, welche sich neben der chemischen Zusammensetzung, in der durchschnittlichen Partikelgröße und dem Vernetzungsgrad der Perlen unterscheiden. Idealer Weise umfasst die Pulverkomponente große Perlen, die gute mechanische Eigenschaften im Prothesenmaterial ergeben, aus mittleren Perlen, die dem System seine Robustheit gegenüber Verfärbungen verleihen sowie einem Anteil kleiner Perlen, die in den Zwischenräumen der anderen Platz finden und die Packungsdichte erhöhen können. Große Perlen führen leicht zu Weißverfärbung und können daher nur zu einem gewissen Anteil verwendet werden. Bei den mittleren Perlen bestimmt sich der Einfluss auf das System über ihr Anquellverhalten. Sehr kleine Perlen quellen üblicher Weise sehr schnell an und erhöhen die Viskosität des angemischten Prothesenmaterials sehr stark, so dass die Gefahr steigt, dass ein korrektes Ausfließen in die Prothesenform nicht mehr gewährleistet ist. Daher ist der ideale Durchmesser der Perlen nach unten ebenfalls beschränkt.

Eine hohe Transparenz kann durch die optimale Auswahl der Rezepturkomponenten in Bezug auf ihre Brechungsindices erreicht werden. Zur Lösung der vorgenannten Aufgaben wurden alle Komponenten hinsichtlich ihres spezifischen Einflusses auf das Reaktionssystem untersucht und so miteinander kombiniert, dass beim Mischen der Pulverkomponenten mit dem ausgewählten Monomer-System die Verarbeitungszeit lang genug ist, um auch Doppelküvetten blasenfrei füllen zu können. Gleichzeitig ist die Temperaturentwicklung so hoch, dass die üblichen Polymerisationszeiten ausreichen und trotz hoher Biegefestigkeit high Impact Werte erreicht werden, die Transparenz sehr gut ist und ein überdurchschnittlich niedriger Rest-MMA-Gehalt resultiert.

Die optimale Kugelpackung zur Erzielung der mechanischen Werte kann durch die Verwendung eines definierten trimodalen Perlensystems erreicht werden, in dem die kleinsten polymeren Partikel, insbesondere Perlen, einen mittleren Durchmesser von ca. 35 Mikrometer aufweisen, die mittleren polymeren Partikel einen mittleren Durchmesser von ca. 45 Mikrometer und die größten polymeren Partikel einen mittleren Durchmesser von ca. 60 µm aufweisen. Vorzugsweise werden die Anteile der drei Perlen synonym Partikelgrößenfraktionen von polymeren Partikeln definiert aufeinander eingestellt. Die optimale Kugelpackung in dem Prothesenmaterial kann überdies weiter verbessert werden durch den Einsatz von polymeren Partikeln mit nanoskaligen Primärpartikeln, welche bevorzugt in der flüssigen Monomerkomponente vorliegen, um eine optimale homogene Verteilung einstellen zu können. Vorzugsweise umfasst mindestens eine Partikelgrößenfraktion Core-Shell-Partikel (Kern-Schale Partikel), um eine noch höhere Bruchsicherheit der polymerisierten Prothese zu erhalten. Die MMA-basierte Monomermatrix wird vorzugsweise durch mindestens 5 Gew.-%, bevorzugt 10 Gew.-% eines kurzkettigen, aliphatischen Acryloyloxy-Ethylcarbamates modifiziert.

Überraschender Weise konnte auf Grund des Austausches von 10 Gew.-% MMA gegen das Carbamat eine Reduktion des Rest-MMA-Gehaltes von mindestens 30 Gew.-% erzielt werden. Bei einer Polymerisationszeit von 30 min im Drucktopf bei 2 bar und einer Wassertemperatur von 55 °C weist das erfindungsgemäße Produkt 48 Stunden nach Herstellung nur noch 2.0 Gew.-% Rest-MMA-Gehalt auf. Der gemessene Wert sinkt nach 4 Tagen auf 1.8 Gew.-%. Die Verminderung des MMA-Gehaltes auf 1.8 Gew.-% kann beschleunigt werden indem die Polymerisation für 60 min durchgeführt wird. In diesem Fall kann der Wert von 1.8 Gew.-% bereits 48 Stunden nach der Herstellung erreicht werden. Da mit diesen Werten selbst die Norm für heißhärtende Materialien weit unterschritten wird, besitzt das erfindungsgemässe Material erstmalig nicht mehr den Nachteil herkömmlicher Kaltpolymerisate mit einem deutlich höheren Rest-MMA-Gehalt. Die bekannten Kaltpolymerisate weisen u.a. aufgrund der kurzen Polymerisationszeit und der milden Polymerisationsbedingungen einen höheren Rest-MMA Gehalt auf.

Das erfindungsgemässe Prothesenbasismaterial weist einen um mindestens 30 Gew.-% niedrigeren Rest-MMA-Gehalt auf, als klassische Kaltpolymerisate und verfügt über Werte für die Brucharbeit von mindestens 1200 J/m² und eine Biegefestigkeit von mindestens 70 MPa sowie über eine Bruchstabilität die den klassischen Kaltpolymerisaten weitaus überlegen ist. Zugleich beträgt vorzugsweise die Transparenz mindestens 95 %.

Es wurde überraschend gefunden, dass der Restmonomergehalt an Methylmethacrylat, weiter deutlich im auspolymerisierten Material nach Normbedingungen gesenkt werden kann, wenn ein Gehalt mindestens eines (ii) N-Alkyl- oder N-Alkenyl-substituiertes Acryloyloxy-Carbamats mit einer Molmasse von kleiner gleich 250 g/mol oder eine Mischung von Carbamaten in der Zusammensetzung eingesetzt wird und die Menge an Carbamat zumindest in etwa eine gleiche Menge an Methylmethacrylat ersetzt. Dabei wird als N-Alkyl- oder N-Alkenyl-substituiertes Acryloyloxy-Carbamat vorzugsweise ein N-Alkyl- oder N-Alkenyl-substituiertes Acryloyloxy-Alkylen-Carbamat (N-Alkyl-Acryloyloxy-(CH₂)ₙ-Carbamat, n = 1, 2, 3, 4, 5, 6) eingesetzt, besonders bevorzugt ein N-Alkyl- oder N-Alkenyl-substituiertes Acryloyloxy-Methylen-Carbamat, Acryloyloxy-Ethylen-Carbamat oder Acryloyloxy-Propylen-Carbamat. Ein N-Alkyl-Acryloyloxy-Ethylen-Carbamat ist besonders bevorzugt. Weiter bevorzugt ist das N-Alkyl substituierte Acryloyloxy-Carbamat ausgewählt aus Methyl,- Ethyl-, n-Propyl-, iso-Propyl, n-Butyl-, i-Butyl-, tert-Butyl, Pentyl- oder Hexyl- und deren Strukturisomeren. Bevorzugte Carbamate sind: isoButyl Acryloyloxy-ethyl-carbamate, tert-Butyl Acryloyloxy-ethyl-carbamate, n-Propyl Acryloyloxy-ethyl-carbamate, n-Propyl Acryloyloxy-propyl-carbamate, wobei erfindungsgemäss n-Butyl Acryloyloxy-ethyl-carbamate (BAEC) eingesetzt werden kann. Gegenstand der Erfindung ist auch die Verwendung von mit mindestens einer elastischen Phase modifizierten Kern-Schale Partikeln und mindestens einem N-Alkyl- oder N-Alkenyl-substituiertes Acryloyloxy-Carbamat mit einer Molmasse von kleiner gleich 250 g/mol, vorzugsweise einem Mono-Carbamat.

Bevorzugt wird eine Monomerkomponente mit einem MMA-Gehalt zwischen 60 bis 95 Gew.-%, vorzugsweise von 60 bis 90 Gew.-%, besonders bevorzugt von 60 bis 85 Gew.-% oder auch 70 bis 90 Gew.-% und einem 5 bis 20 Gew.-%igen Anteil eines aliphatischen Acryloyloxy-Carbamats mit einer Molmasse kleiner 250 g/mol eingesetzt. Zudem wird bevorzugt eine Mischung von polymeren Partikeln mit mindestens drei verschiedenen Partikelgrößenfraktionen von PMMA-basierten polymeren Partikeln bzw. Perlen die sich in ihrem durchschnittlichen Durchmesser voneinander um mindestens 5 µm unterscheiden.

Entsprechend einer besonders bevorzugten Alternative umfasst das Prothesenbasismaterial die Komponente (A) und (B), wobei das Prothesenbasismaterial in der Gesamtzusammensetzung in Gewichtsprozent ad 100 Gew.-% umfasst:
(i) 20 bis 50 Gew.-% Methylmethacrylat (MMA),
(ii) 1 bis 30 Gew.-% mindestens ein N-Alkyl- oder N-Alkenyl-substituiertes Acryloyloxy-Carbamat mit einer Molmasse von kleiner gleich 250 g/mol,
(iii) 0,5 bis 10 Gew.-% mindestens ein mindestens difunktionelles Urethan(meth)acrylat, wie Oligomer oder auch Dendrimer,
(iv) 0,05 bis 10 Gew.-% mindestens ein di-, tri-, tetra- oder multi-funktionelles Monomer, das kein Urethan(meth)acrylat ist,
(v) 0,1 bis 10 Gew.-% polymere Partikel, die als durch eine elastischen Phase modifizierte Kern-Schale-Partikel vorliegen, mit einer Primärpartikelgröße von kleiner 800 nm,
(vi) 0,05 bis 2 Gew.-% mindestens einen Initiator oder mindestens eine Komponente eines Initiatorsystems für die Autopolymerisation, sowie
(vii) 48,3 bis 78,3 Gew.-%, insbesondere 55 bis 65 Gew.-%, mindestens eine pulverförmige Komponente (B) die polymere Partikel enthält, die mindestens drei verschiedene Fraktionen von Partikelgrößen polymerer Partikel umfasst, wobei sich die Angaben in Gew.-% auf die Gesamtzusammensetzung beziehen, und wobei die drei Fraktionen ausgewählt sind aus polymeren Partikeln einer mittleren Partikelgröße
   a) von 25 bis kleiner 40 µm, die zu 50 bis 90 Gew.-%,
   b) von 40 bis kleiner 55 µm, die zu 0,1 bis 20 Gew.-%, und c) von 55 bis 100 µm, die zu 0,5 bis 30 Gew.-% vorliegen, wobei sich die Angaben von a), b) und c) in Gew.-% auf die Gesamtzusammensetzung der Komponente (B) beziehen.

Gleichfalls Gegenstand der Erfindung ist das auspolymerisierte Prothesenbasismaterial das einen Restmonomergehalt an MMA von kleiner gleich 3 Gew.-% bestimmt nach ISO 20795-1:2013 aufweist, insbesondere weist es einen Restmonomergehalt an MMA von kleiner gleich 2,5 Gew.-%, bevorzugt von kleiner gleich 2,4, 2,3, 2,2, 2,1, 2,05, 2,0 oder 1,9 Gew.-% auf, insbesondere mit einer Schwankungsbreite von +/- 0,05 Gew.-%.

Ferner ist Gegenstand der Erfindung das auspolymerisierte Prothesenbasismaterial, das eine Transparenz von größer gleich 95 % (gemessen an 3 mm dicken Farbprüfkörpern hergestellt in Metallformen mittels Farbmessgerät SF 600 (Datacolor)) aufweist, insbesondere grösser gleich 97 %.

Nach einer weiteren Alternative ist Gegenstand der Erfindung das auspolymerisierte Prothesenbasismaterial, das eine Bruchzähigkeit als Höchstfaktor der Beanspruchungsintensität Kmax von ≥ 2,4 Mpa m^{1/2}, insbesondere grösser gleich 2,45 Mpa m^{1/2} bevorzugt grösser gleich 2,5 Mpa m^{1/2}, und eine Bruchzähigkeit als Gesamtbrucharbeit Wf (J/m²) von ≥ 1000 J/m² aufweist, insbesondere von größer gleich 1100 J/m², bevorzugt von grösser gleich 1200 J/m²., weiter bevorzugt von grösser gleich 1250 J/m². Besonders bevorzugt ist zudem die Biegefestigkeit grösser 65 MPa, besonders bevorzugt grösser 70 MPa. Weiter ist es bevorzugt, wenn die Transparenz der unpigmentierten, auspolymerisierten Prothesen im Bereich von grösser gleich 90 %, insbesondere grösser gleich 95 % liegt (gemessen an 3 mm dicken Platte).

Erfindungsgemäss werden die Aufgaben durch die synergistische Verwendung des Carbamates mit einem Molekulargewicht von kleiner gleich 250 g/mol, der Verwendung von mindesten einer oder mehreren Fraktionen an Kern-Schale-Partikeln mit einem elastischen Kern sowie mindestens drei verschiedenen Fraktionen an Partikelgrössen der polymeren Partikel in der pulverförmigen Komponente gelöst. Die hohe Transparenz des Prothesenbasismaterials konnte durch die Auswahl spezifischer Kern-Schale Partikel mit einer Brechzahl ähnlich der des auspolymerisierten Prothesenwerkstoffes gewährleistet werden. Daher liegen die polymeren Partikel mit einer Partikelgröße von kleiner 800 nm vorzugsweise als mit einer elastischen Phase modifzierte Kern-Schale Partikel vor und weisen vorzugsweise eine Brechzahl von etwa 1,49 auf (R.I. ∼ 1,4900).

Erfindungsgemäss besonders bevorzugte Kern-Schale-Partikel liegen aggregiert vor. Die Aufgaben können durch die Verwendung von aggregierten Kern-Schale Partikeln (unregelmässig geformte Aggregate, d₅₀ ∼ 50 - 300 µm) gelöst werden, die Primärpartikelgrösse beträgt ca. 200 - 400 nm. Die Kern-Schale-Partikel liegen vermutlich aufgrund von Oberflächenwechselwirkungen im Feststoff aggregiert vor. Das Additiv wird mit der Flüssigkeit gemischt und dispergiert und bildet eine stabile Suspension, welche innerhalb von wenigen Wochen nur schwach sedimentiert. Durch die Suspendierung in MMA zerfallen die Aggregate relativ rasch in die Primärpartikel.

Durch die Verwendung der Kern-Schale-Partikel als High-Impact Additiv in Kombination mit mindestens einem Carbamat mit einem Molekulargewischt kleiner 250 g/mol vorzugsweise n-Butylacryloxyethyl-Carbamat, lassen sich Prothesenbasismaterialien herstellen, welche die Anforderungen der ISO 20795-1 hinsichtlich High-Impact Eigenschaften erfüllen. Darüber hinaus kann ein Prothesenwerkstoff bereitgestellt werden, dessen Biegefestigkeit und E-Modul in der gleichen Grössenordnung wie Nicht-High-Impact Werkstoffe liegt und gleichzeitig hochtransparent und farbstabil ist. Die erfindungsgemässen Prothesen zeigen keine oder kaum Weissverfärbungen durch Kontakt mit wasserhaltigen Materialien wie z.B. Dubliergele oder Gipse während und nach dem Polymerisationsprozess.

Gegenstand der Erfindung ist auch ein Prothesenbasismaterial das jeweils unabhängig die Komponente (A) umfasst, welche (v) polymere Partikel enthält, die als durch eine elastische Phase modifizierte Kern-Schale-Partikel vorliegen, wobei die elastische Phase als Kern in härterer Aussenschale (Kern-Schale-Teilchen) vorliegt, und insbesondere die elastische Phase Styrol-Butylacrylat-Polymere umfasst, und/oder unabhängig die Komponente (B) umfasst mindestens eine Fraktion polymerer Partikel, die als durch eine elastische Phase modifizierte Kern-Schale Partikel vorliegen, wobei die elastische Phase als Kern in härterer Aussenschale (Kern-Schale-Teilchen) vorliegt, und insbesondere die elastische Phase Styrol-Butylacrylat-Polymere umfasst. Die Kern-Schale Partikel umfassen in der Regel (unregelmässig geformte Aggregate, d₅₀ ∼ 50 - 300 µm), die in der Monomerkomponete zu den Primärpartikeln gelöst werden. Die Primärpartikelgrösse beträgt ca. 200 - 400 nm. Die Kern-Schale-Partikel liegen vermutlich aufgrund von Oberflächenwechselwirkungen im Feststoff aggregiert vor.

Ferner kann der erfindungsgemässe Prothesenwerkstoff mit üblichen zahntechnischen Methoden und Instrumenten gemischt und verarbeitet werden. Zur Verarbeitung können herkömmliche, dem Zahntechniker gebräuchliche Techniken und Hilfsmittel verwendet werden. Spezielle Einbettmassen, Gipse, Küvetten, Geräte etc. (wie z.B. beim Ivoclar-System) können vermieden werden.

Zur Abgrenzung von Prothesenmaterialien zu üblichen dentalen Materialien wird hervorgehoben, dass Prothesenmaterialien wesentliche Mengen an polymeren pulverförmigen Komponenten umfassen, wie PMMA (Poly(meth)methylacrylat) und/oder (Poly(ethyl)-methacrylat, insbesondere zu grösser gleich 50 Gew.-% in der Gesamtzusammensetzung. Übliche Prothesenmaterialien werden in der Regel in einem Kit mit einer pulverförmigen Komponente und einer flüssigen Komponente angeboten. Dentale Materialien zur Herstellung von Füllungen basieren im Wesentlichen auf anorganischen Füllstoffen (Dentalgläsern) die vorzugsweise mit einem Anteil von grösser 60 Gew.-% in den polymerisierbaren Zusammensetzungen vorliegen sowie hauptsächlich hochmolekularer Monomere, die häufig auf BisGMA (Bisphenol-A-(di)-methacrylat) basieren.

Das mindestens eine difunktionelle oder mehrfachfunktionelle Urethan(meth)acrylat kann ausgewählt werden aus einem Urethandimethacrylat, bevorzugt einem Bis(methacryloxy-2-ethoxycarbonylamino)-alkylen, Acryloxy substituiertes Urethan-Dentrimer, Diurethanacrylat Oligomer, Urethan(meth)acrylat Dendrimere, Urethane methacrylate polymer ca. 300 g/mol (Dendrimer, Laromer UA9049, 50 % in Acrylat Monomerblend (41,7 Gew.-% HEMA-TMDI / 8Gew.-% TEGDMA, CAS 109-16-0), Alkyl-funktionelle Urethandimethacrylat Oligomere, Aromatisch-funktionalisierte Urethandimethacrylat Oligomere, aliphatische ungesättigte Urethanacrylate, Bis(methacryloxy-2-ethoxycarbonylamino) substituierter Polyether, aromatische Urethandiacrylat Oligomere, aliphatische Urethandiacrylat Oligomere, monofunktionelle Urethanacrylate, aliphatische Urethandiacrylate, hexafunktionelle aliphatische Urethanharze, aliphatisches Urethantriacrylat, UDMA, aliphatisches Urethanacrylat Oligomer, ungesättigte aliphatische Urethanacrylate. Geeignete Urethan(meth)acrylate sind unter den folgenden Markennamen erhältlich: Ebecryl 230 (aliphatisches Urethandiacrylat), Actilane 9290, Craynor 9200 (Di-Urethanacrylat Ooligomer), Ebecryl 210 (aromatische urethan-diacrylat Oligomere), Ebecryl 270 (aliphatische Urethandiacrylat Oligomer), Actilane 165, Actilane 250, Genomer 1122 (monofunktionelles Urethan-acrylat), Photomer 6210 (cas no. 52404-33-8, aliphatisches Urethan-diacrylat), Photomer 6623 (hexafunctional aliphatic Urethan Resin), Photomer 6891 (aliphatisches urethan-triacrylat), UDMA, Roskydal LS 2258 (Aliphatisches Urethan-acrylat Oligomer), Roskydal XP 2513 (ungesättigtes aliphatisches Urethanacrylat). Dabei ist es besonders bevorzugt, wenn die Komponente (v), die durch mindestens eine elastische Phase modifizierten Kern-Schale-Partikel zu 0,001 bis 20 Gew.-% vorliegen, insbesondere bis 10 Gew.-%, bevorzugt bis 5 Gew.-%, in Bezug auf die Gesamtzusammensetzung der Komponente (A) und (ii) mindestens ein Carbamat mit einer Molmasse kleiner 250 g/mol, insbesondere kein Diurethan, von 0,001 bis 30 Gew.-%, vorzugsweise bis 10 Gew.-%, weiter bevorzugt bis 5 Gew.-%, in Bezug auf die Gesamtzusammensetzung der Komponente (A) vorliegt (d.h. auf 100 Gew.-% der Komponente (A)). Die Kern-Schale-Partikel werden auch als High-Impact Modifizierer bezeichnet.

Das erfindungsgemässe Prothesenbasismaterial besteht trotz des zugesetzten High Impact-Modifizierers (Kern-Schale-Partikel)und des Carbamates den Suntests nach ISO 20795-1.

Besonders bevorzugte Prothesenbasismaterialien weisen vorzugsweise Kern-Schale-Partikel auf, in denen die Verteilung der elastischen Phase der modifizierten Kern-Schale-Partikel ausgewählt ist aus den Möglichkeiten a bis d: a) elastische Phase als Kern (z.B. aus Butylacrylat) in harter Aussenschale (z.B. aus PMMA) (Kern-Schale-Teilchen); b) mehrere elastische Phasen als Kerne in einer harten Matrix, c) Kern-Schale-Teilchen aus a), in harter Matrix verteilt und d) harter Kern mit elastischer Phase als Aussenschale. Erfindungsgemässe Kern-Schale-Partikel könne ebenso den nachfolgenden multi-layer Aufbau aufweisen, e) einen innen liegenden Kern mit mehreren Schichten als Schalen und einer Aussenschale, wobei insbesondere (i) mindestens eine der Schalen, vorzugsweise die Aussenschale hart ist und die verbleibenden Schalen und der Kern jeweils unabhängig aus elastischen Phasen bestehen. In Alternativen können die elastischen Phasen und die harten Phasen auf die Schalen und den Kern anderweitig aufgeteilt sein.

Ferner weisen bevorzugte Kern-Schale-Partikel eine Brechzahl ähnlich der des auspolymerisierten Prothesenwerkstoffes auf. Vorzugweise liegt die Brechzahl der Kern-Schale Partikel um 1,49 mit einer Schwankungsbreite von plus/minus 0,02, insbesondere +/- 0,01. Erfindungsgemäss besonders bevorzugte Kern-Schale-Partikel liegen aggregiert vor. Dabei weisen die Aggregate der Kern-Schale Partikel, die regellos geformt sein können, als unregelmässig geformtes Aggregat einen mittleren Durchmesser d₅₀ ∼ 50 - 300 µm auf. Die bevorzugte Grösse der Primärteilchen beträgt kleiner 500 nm, insbesondere bis 100 nm, bevorzugt von 200 bis 400 nm. Gleichfalls können Kern-Schale-Partikel mit einer Primärpartikelgrösse von kleiner gleich 200 nm bis 2 nm, sowie zwischen 150 bis 10 nm als Kern-Schale Partikel eingesetzt werden.

Vorzugsweise weisen die Kern-Schale Partikel einen Brechungsindex von 1,48 bis 1,60 auf, insbesondere von 1,49 bis 1,55. Besonders bevorzugt liegt der Brechungsindex der Kern-Schale Partikel im Bereich des Brechungsindexes von PMMA, vorzugsweise liegt der Brechungsindex daher um 1,48 bis 1,50.

Gleichfalls bevorzugt sind Kern-Schale Partikel deren Dichte bei 0,9 bis 1,5 g/ml, insbesondere von 0,95 bis 1,4 g/ml liegt. Vorzugsweise liegt die Schüttdichte zugleich bei 0,1 bis 0,6 g/ml, bevorzugt bei 0,1 bis 0,6 g/ml.

Unter einer harten Aussenschale, harten Matrix, hartem Kern wird ein Material verstanden, das vorzugsweise eine geringere Elastizität als das Material der elastischen Phase aufweist. Bevorzugte anorganische harte Kerne zeigen unter dem Einfluss einer Kraft im Wesentlichen keine Verformung, während die organischen harten Materialien unter Einwirkung einer Kraft eine deutlich geringere Verformung erleiden als die elastische Phase. Die harten Materialien als harte Aussenschale, harte Matrix und/oder harter Kern stabilisieren die elastische Phase in ihrer Form. Eine elastische Phase wird aus mindestens einem elastischen Material gebildet, das unter der Einwirkung einer Kraft eine reversible Verformung erfährt. Die Verformung der elastischen Phase ist vorteilhaft ohne Krafteinwirkung vollständig reversibel.

Bevorzugte Komponenten B) umfassen vorzugsweise mindestens eine pulverförmige Komponente umfassend a) polymere Partikel umfassend Polymere in Form von Polymerpulver umfassend Polyalkyl(meth)acrylate, die optional vernetzt sind und als Homo- oder Co-Polymere vorliegen, wobei die Polymere auf mindestens einem der Monomere, umfassend eine (Meth-) acrylat-Gruppe basieren, ausgewählt aus Methylmethacrylat, Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Polypropylenglykol-mono-acrylat, Hydroxyethylacrylat, Hydroxypropylacrylat, Hydroxyethylmethacrylat, Hydroxypropylmethacrylat, eine Mischung enthaltend mindestens eines dieser (Meth-)acrylate und/oder Co-Polymere umfassend eines oder mindestens zwei der vorgenannten Monomere, Polyamidpartikel, Polyamidfasern. Darüber hinaus können die polymeren Partikel auch Gemische dentaler Monomeren wie z.B. MMA und zusätzlich mindestens einen Vernetzer umfassen. Entsprechend einer bevorzugten Ausführungsvariante der Erfindung umfasst jeweils unabhängig eine Partikelgrößenfraktion polymerer Partikel der pulverförmigen Komponente (B) Polymethylmethacrylat (PMMA) Partikel bzw. Perlen. Besonders bevorzugt umfasst die pulverförmige Komponente Polymethylmethacrylat (PMMA) Perlen als polymere Partikel und/oder Splitterpolymerisate, insbesondere mit Teilchengrössen von 10 - 100 µm, und/oder basierend auf Co-Polymeren umfassend einpolymerisierte Comonomere Styrol, alpha-Methylstyrol, Vinyltoluol, substituierte Vinyltoluole wie Vinylbenzylchloride, Vinylhalogenide wie Vinylchlorid, Vinylester, wie Vinylacetat, heterocyclische Vinylverbindungen wie 2-Vinylpyridin, Vinylacetat und Vinylpropionat, Butadien, Isobutylen, 2-Chlorbutadien, 2-Methylbutadien, Vinylpyridin, Cyclopenten, (Meth)acrylsäureester, wie Methylmethacrylat, Ethylmethacrylat, Butylmethacrylat, Butylacrylat und Hydroxyethylmethacrylat, ferner Acrylnitril, Maleinsäure und Maleinsäurederivate, wie beispielsweise Maleinsäureanhydrid, Fumarsäure und Fumarsäurederivate wie Fumarsäureester, Acrylsäure, Methacrylsäure sowie Aryl(meth)acrylate wie Benzylmethacrylat oder Phenylmethacrylat sowie optional Mischungen dieser Comonomere und optional zusätzlich b) anorganische Füllstoffe umfassend pyrogene oder Fällungskieselsäuren, Dentalgläser wie Aluminosilicatgläser oder Fluoroaluminosilicatgläser, Bariumaluminiumsilicat, Strontiumsilicat, Strontiumborosilicat, Lithiumsilicat, Lithiumaluminiumsilicat, Schichtsilikate, Zeolithe, amorphe sphärische Füller auf Oxid- oder Mischoxidbasis, insbesondere Mischoxide von SiO₂ und ZrO₂, Glasfasern und/oder Kohlenstofffasern sowie Mischungen umfassend die pulverförmigen Komponenten a) und b).

Die b) anorganischen Füllstoffe werden in der Regel zu 0 bis 10 Gew.-%, vorzugsweise von 0,0001 bis 3 Gew.-%, bezogen auf die gesamte Prothesenkunststoffzusammensetzung bzw. die Summe der Komponenten (A) und (B) eingesetzt. In der Komponente (B) in Bezug auf die Gesamtzusammensetzung der Komponente (B) von 100 Gew.-% liegen sie in der Regel im Bereich von 0 bis 20 Gew.-% vor, vorzugsweise 0,001 bis 10 Gew.-%.

Ebenfalls im Sinne der Erfindung sind polymere Partikel, die auf mindestens einem (Meth-)-acrylat Monomer mit nur einer (Meth-)acrylat-Gruppe basieren oder die auf der Mischung mindestens zweier dieser (Meth-)acrylat Monomere basiert.

Erfindungsgemässe Kern-Schale-Partikel umfassen als elastische Phase vorzugsweise mindestens ein Poly-(n-butyl-acrylat) PBA, besonders bevorzugt Styrol-Butylacrylat-Polymere. Ebenso geeignet sind andere Butadien-Styrol-Copolymere, Nitril-Butadien-Copolymer, Silikonkautschuk-(Pfropfcopolymerisat), Polyurethanpolymerisat, Polyolefin-basiertes Polyurethan (Polybutadien-basiertes Polyurethan), das vorzugsweise in MMA vorliegen kann. Die Partikelgrösse der Kern-Schale-Partikel kann kleiner gleich 500 nm, wie zwischen 50 nm bis 500 nm, insbesondere kleiner gleich 400 nm bis 100 nm, oder alternativ kleiner 100 nm bis 2 nm betragen, gleichfalls kann die elastische Phase auf Polydimethylsiloxan modifiziertem Polyurethanen und/oder Epoxy-funktionalisierten elastischen Phasen basieren.

Erfindungsgemässe Kern-Schale-Partikel umfassen als harte Schale, harten Kern und/oder harte Matrix mindestens ein (Meth)acrylat Polymer, vorzugsweise ein Alkyl(meth)acrylatPolymer, wie PMMA; Polystyrol, einem Epoxy-funktionalisierten Kern, sowie Homo- oder Co-Kondensate der vorgenannten Polymere.

Bevorzugte Kern-Schale-Partikel umfassen Aggregate mit d₅₀ < 400 µm und Primärpartikelgrössen von d₅₀ kleiner 500 nm. Weiter bevorzugt können die Primärpartikel der Kern-Schale Partikel grösser gleich 100 nm sein, insbesondere als d₅₀-Wert.

Gleichfalls geeignete Kern-Schale Partikel umfassen einen elastischen Kern umfassend Acrylat-Polymere mit harter Aussenschale, insbesondere mit einer Partikelgrösse kleiner 1 Mikrometer. Weiter können die Kern-Schale-Partikel bevorzugt gegenüber polymerisierbaren Monomeren reaktive Gruppen aufweisen, vorzugsweise ist die Aussenschale mit (Meth)acrylatGruppen funktionalisiert.

Ferner ist Gegenstand der Erfindung ein Prothesenbasismaterial umfassend eine (A) flüssige Monomerkomponente, die mindestens ein Monomer, insbesondere eine Mischung von Monomeren umfasst von Methylmethacrylat sowie optional mindestens einem (2-Alkyl)-Acrylsäureester, insbesondere umfasst die Komponente (A) Methylmethacrylat. In einer Alternative kann die Komponente (A) mindestens eines der folgenden Monomere oder eine Mischung umfassend mindestens zwei der genannten Monomere enthalten: Methylmethacrylat sowie optional zusätzlich Ethylmethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylacrylat, Propylacrylat, Butylacrylat, n-Hexylacrylat, 2-Phenoxyethylacrylat, Isobornyl-acrylat, Isodecylacrylat, Polypropylen-glykol-mono-acrylat, Tetrahydrofuryl-acrylat, Polypropylen-glykol-mono-acrylat, Hydroxyethylacrylat, Hydroxypropylacrylat, Hydroxyethylmethacrylat, Hydroxypropylmethacrylat, Benzyl-, Furfuryl- oder Phenyl(meth)acrylat eine Mischung enthaltend mindestens eines dieser (Meth-)acrylate und/oder Co-Polymere umfassend eines oder mindestens zwei der vorgenannten Monomere.

Ferner umfasst die Komponente (A) mindestens ein (iv) di-, tri-, tetra- oder multi-funktionelles Monomer, das kein Urethan(meth)acrylat ist, wie vorzugsweise Tris-(2-Hydroxyethyl)-isocynaurat-Triacrylat (Sartomer 368). Alternativ kann als (iv) di-, tri-, tetra- oder multi-funktionelles Monomer, das kein Urethan(meth)acrylat ist, eines der folgenden Monomere oder eine Mischung umfassend mindestens zwei der genannten Monomere eingesetzt werden: 1,4-Butandiol-dimethacrylat (1,4-BDMA) oder Pentaerythritol-tetraacrylat, Bis-GMA-Monomer (Bisphenyl-A-Glycidyl-Methacrylat), Triethylen-glycoldimethacrylat (TEGDMA) und Diethylenglycoldimethacrylat (DEGMA), Tetraethylen-glykoldi(meth)acrylat, Decandioldi(meth)acrylat, Dodecandioldi(meth)acrylat, Hexyldecan-dioldi(meth)acrylat, Trimethylolpropantri(meth)acrylat, Pentaerythrittetra(meth)-acrylat sowie Butandioldi(meth)acrylat, Ethylenglycol-di(meth)acrylat, Polyethylenglycol-di(meth)acrylate, ethoxylierte/propoxylierte Bisphenol-A-di(meth)acrylate eine Mischung enthaltend mindestens eines dieser (Meth-)acrylate und/oder Co-Polymere umfassend eines oder mindestens zwei der vorgenannten Monomere. Als geeignete Alkylmethacrylate für die flüssige Komponente (A) sei auf Methyl-, Ethyl-, n-Propyl, i-Propyl-, n-Butyl, t-Butyl, i-Butyl, Benzyl- und Furfurylmethacrylate oder deren Mischungen verwiesen. Methylmethacrylat ist davon besonders bevorzugt.

Vorzugsweise umfasst die (A) flüssige Monomerkomponente
(i) 60 bis 90 Gew.-%, bevorzugt zu 60 bis 85 Gew.-% Methylmethacrylat sowie optional mindestens ein (2-Alkyl)-Acrylsäureester, der nicht MMA ist,
(ii) 5 bis 20 Gew.-% mindestens ein N-Alkyl- oder N-Alkenyl-substituiertes Acryloyloxy-Carbamat mit einer Molmasse von kleiner gleich 250 g/mol,
(iii) 0,5 bis 10 Gew.-% mindestens ein mindestens difunktionelles Urethan(meth)acrylat,
(iv) 0,05 bis 10 Gew.-% mindestens ein di-, tri-, tetra- oder multi-funktionelles Monomer, das kein Urethan(meth)acrylat ist,
(v) 0,1 bis 10 Gew.-% polymere Partikel, die als durch eine elastischen Phase modifizierte Kern-Schale-Partikel vorliegen, mit einer Primärpartikelgröße von kleiner 800 nm,
(vi) 0,05 bis 2 Gew.-% mindestens einen Initiator oder mindestens eine Komponente eines Initiatorsystems für die Autopolymerisation, wobei sich die vorstehenden Angaben in Gew.-% auf die Gesamtzusammensetzung der Komponente (A) beziehen.

Die Kern-Schale-Partikel werden in der Regel in den Monomeren suspendiert.

Erfindungsgemäss setzt sich die Gesamtzusammensetzung der pulverförmigen Komponente (B) wie folgt zusammen:
(i) 90 bis 99,95 Gew.-% mindestens eine pulverförmige Komponente polymerer Partikel, die mindestens drei verschiedene Fraktionen von Partikelgrößen polymerer Partikel umfasst, wobei die drei Fraktionen ausgewählt sind aus polymeren Partikeln einer mittleren Partikelgröße a) von 25 bis kleiner 40 µm, die zu 50 bis 90 Gew.-%, b) von 40 bis kleiner 55 µm, die zu 0,1 bis 20 Gew.-%, und c) von 55 bis 100 µm, die zu 0,5 bis 30 Gew.-% vorliegen, und
(ii) 0,05 bis 10 Gew.-% mindestens einen Initiator oder mindestens eine Komponente eines Initiatorsystems für die Autopolymerisation, wobei sich die vorstehenden Angaben in Gew.-% auf die Gesamtzusammensetzung der Komponente (B) beziehen.

Die Gesamtzusammensetzung (A) mit 100 Gew.-% und die Gesamtzusammensetzung (B) mit 100 Gew.-% können im Gewichtsverhältnis von (A) zu (B) von 1 zu 20 bis 20 zu 1 gemischt werden, bevorzugt im Gewichtsverhältnis von (A) zu (B) von 1 : 10 bis 10 : 1, bevorzugt von 5 zu 15 bis 9 zu 8, bevorzugt von 5 bis 8 zu 8 bis 12, vorzugsweise im Gewichtsverhältnis von (A) zu (B) von 7 zu 10, insbesondere mit einer Schwankungsbreite von plus/minus 1, vorzugsweise von 0,5.

Der Anteil an bei Raumtemperatur flüssigem Methylmethacrylat im erfindungsgemässen, gemischten, noch nicht auspolymerisiertem Prothesenbasismaterial, insbesondere umfassend die Komponenten (A) und (B), beträgt insbesondere 20 Gew.-% bis 50 Gew.%, bevorzugt 30 bis 40 Gew.%.

Ferner ist Gegenstand der Erfindung ein Prothesenbasismaterial, das vorzugsweise in Komponente (A), (B) oder in (A) und (B) zusätzlich mindestens einen oder mehrere Stoff(e) aus den Gruppen der Füllstoffe, Pigmente, Stabilisatoren, Regler, antimikrobiellen Additive, UV-Absorber, Thixotropiermittel, Katalysatoren und Vernetzer enthält. Solche Additive werden - wie auch Pigmente, Stabilisatoren und Regler - in eher geringen Mengen eingesetzt, z. B. insgesamt zu 0,01 bis 3,0, besonders 0,01 bis 1,0 Gew.% bezogen auf die Gesamtmasse des Materials. Geeignete Stabilisatoren sind z. B. Hydrochinonmonomethylether oder 2,6-Di-tert.-butyl-4-methylphenol (BHT).

Gleichfalls Gegenstand der Erfindung ist ein Prothesenbasismaterial das optional zusätzlich mindestens einen Initiator oder mindestens ein Initiatorsystem für die Autopolymerisation aufweist, das je nach Reaktionsbedingungen bzw. Polymerisationssystem, in der flüssigen Komponente (A), der pulverförmigen Komponente (B) oder in (A) und (B) vorliegen kann.

Die folgenden Initiatoren und/oder Initiatorsysteme für die Auto- oder Kaltpolymerisation umfassen a) mindestens einen Initiator insbesondere mindestens ein Peroxid und/oder Azoverbindung, insbesondere LPO: Dilauroylperoxid, BPO: Dibenzoylperoxid, t-BPEH: tert.-Butylper-2-ethylhexanoat, AIBN: 2,2'-Azobis-(isobutyronitril), DTBP: Di-tert.-butylperoxid, und optional b) mindestens einen Aktivator, insbesondere mindestens ein aromatisches Amin, wie N,N-Dimethyl-p-toluidin, N,N-Dihydroxyethyl-p-toluidin und/oder p-Dibenzylaminobenzoesäurediethylester oder c) mindestens ein Initiatorsystem ausgewählt aus Redoxsystemen, insbesondere eine Kombinationen ausgewählt aus Dibenzoylperoxid, Dilauroylperoxid und Campherchinon mit Aminen ausgewählt aus N,N-Dimethyl-p-toluidin, N-N-Dihydroxyethyl-p-toluidin und p-Dimethylaminobenzoesäurediethylester oder ein Redoxsystem umfassend ein Peroxid, und ein Reduktionsmittel ausgewählt aus Ascorbinsäure, Ascorbinsäurederivat, Barbitursäure oder ein Barbitursäurederivat, Sulfinsäure, Sulfinsäurederviat, besonders bevorzugt ist ein Redoxsystem umfassend (i) Barbitursäure oder Thiobarbitursäure oder ein Barbitursäure- oder Thiobarbitursäurederivat und (ii) mindestens ein Kupfersalz oder Kupferkomplex und (iii) mindestens eine Verbindung mit einem ionischen Halogenatom, besonders bevorzugt ist ein Redoxsystem umfassend 1-Benzyl-5-Phenylbarbitursäure, Kupferacetylacetonat, Triazin-Derivat, Toluidin-Derivat und/oder Benzyldibutylammoniumchlorid. Besonders bevorzugt wird die Polymerisation im 2-Komponenten Prothesenbasismaterial über ein Barbitursäurederivat gestartet.

Gegenstand der Erfindung ist auch ein Verfahren zur Herstellung eines auspolymerisierten Prothesenbasismaterials, sowie ein Prothesenbasismaterial erhältlich nach dem Verfahren, indem
die Komponenten A) mindestens eine flüssige Monomerkomponente, und B) mindestens eine pulverförmige Komponente, gemischt werden und nachfolgend auspolymerisiert bzw. gehärtet werden. Dabei ist es besonders bevorzugt, wenn die Monomerkomponente (A) und die pulverförmige Komponente (B) im Gewichtsverhältnis von 1 : 10 bis 10 : 1, insbesondere im Gewichtsverhältnis 8 bis 12 pulverförmige Komponente zu 4 bis 8 Monomerkomponente gemischt werden. Bevorzugt 7 zu 10 Komponente (A) zu (B), wobei die Schwankungsbreite +/- 1, vorzugsweise +/- 0,5 betragen kann.

Gleichfalls Gegenstand der Erfindung ist ein Verfahren indem die Monomerkomponente (A) und die pulverförmige Komponente (B) gemischt werden, und insbesondere als auspolymerisierbares Prothesenbasismaterial in eine Negativform überführt wird, wie eine Gussform, mindestens eines dentalen, prothetischen Formkörpers, wie Zahns, Aufbissschiene, Fräsrohlings bzw. Fräsronde, Dentalprothese, Teil von Prothese, Bohrschablone, Implantat, Mouthguard, Gelenkprothesen, Krone, Teleskop, Veneer, Zahnbrücke, Prothesenzahn, Implantateil, Abutment, Suprastruktur, kieferorthopädischer Apparat und Instrument, Hufteil und, insbesondere das Material unter erhöhtem Druck, insbesondere grösser gleich 2 bar, wie 2,5 bis 10 bar, vorzugsweise 2 bis 4 bar, auspolymerisiert wird. Vorzugsweise erfolgt die Polymerisation bei einer Temperatur von 35 °C bis 60 °C, vorzugsweise von 45 °C bis 60 °C, bevorzugt bei circa 55 °C von 20 bis 180 Minuten, bevorzugt bei circa 55 °C für 30 Minuten. Die Polymerisation erfolgt dabei vorzugsweise unter leicht erhöhtem Druck zwischen 1 bis 5 bar, insbesondere bei 2 bar.

Das Mischen der Komponenten A) und B) kann erfindungsgemäss mittels einfacher dem Zahntechniker bekannter Massnahmen erfolgen, wie mittels Spatel.

Nach einer weiteren Alternative ist Gegenstand der Erfindung ein Kit umfassend ein autopolymerisierbares Prothesenbasismaterial, wobei das Kit separierte Komponenten (A) und (B) umfasst, dadurch gekennzeichnet, dass die Komponente (A) umfasst
(i) 60 bis 85 Gew.-% Methylmethacrylat,
(ii) 5 bis 20 Gew.-% mindestens ein N-Alkyl- oder N-Alkenyl-substituiertes Acryloyloxy-Carbamat mit einer Molmasse von kleiner gleich 250 g/mol,
(iii) 0,5 bis 10 Gew.-% mindestens ein mindestens difunktionelles Urethan(meth)acrylat,
(iv) 0,05 bis 10 Gew.-% mindestens ein di-, tri-, tetra- oder multi-funktionelles Monomer, das kein Urethan(meth)acrylat ist,
(v) 0,1 bis 10 Gew.-% polymere Partikel, die als durch eine elastischen Phase modifizierte Kern-Schale-Partikel vorliegen, mit einer Primärpartikelgröße von kleiner 800 nm,
(vi) 0,05 bis 2 Gew.-% mindestens einen Initiator oder mindestens eine Komponente eines Initiatorsystems für die Autopolymerisation, wobei sich die vorstehenden Angaben in Gew.-% auf die Gesamtzusammensetzung der Komponente (A) beziehen, sowie als
Komponente (B) umfassend
(i) 90 bis 99,95 Gew.-% mindestens eine pulverförmige Komponente polymerer Partikel, die mindestens drei verschiedene Fraktionen von Partikelgrößen polymerer Partikel umfasst, wobei die drei Fraktionen ausgewählt sind aus polymeren Partikeln einer mittleren Partikelgröße
   a) von 25 bis kleiner 40 µm, die 20 bis 90 Gew.-%, bevorzugt zu 50 bis 90 Gew.-%,
   b) von 40 bis kleiner 55 µm, die 0,1 bis 50 Gew.-%, bevorzugt zu 0,1 bis 20 Gew.-%, und
   c) von 55 bis 100 µm, die zu 0,5 bis 50 Gew.-%, bevorzugt 0,5 bis 30 Gew.-% vorliegen, und
(ii) 0,05 bis 10 Gew.-% mindestens einen Initiator oder mindestens eine Komponente eines Initiatorsystems für die Autopolymerisation, wobei sich die vorstehenden Angaben in Gew.-% auf die Gesamtzusammensetzung der Komponente (B) beziehen.

Gegenstand der Erfindung ist auch ein auspolymerisiertes Prothesenbasismaterial oder die Verwendung des Prothesenbasismaterials zur Herstellung der folgenden Erzeugnisse in Form eines prothetischen Formkörpers, im humanen Dentalbereich, wie dentalen prothetischen Formkörpers, wie Zahn, Aufbissschiene, Fräsrohlings bzw. Fräsronde, Dentalprothese, Teil von Prothese, Bohrschablone, Implantat, Mouthguard, Gelenkprothesen, Krone, Teleskop, Veneer, Zahnbrücke, Prothesenzahn, Implantateil, Abutment, Suprastruktur, kieferorthopädischer Apparat und Instrument, prothetische Formkörper in Form von Knochen oder Teilen davon, prothetischer Formkörper im Veterinärbereich, wie Hufteil, insbesondere für Hufreparaturmaterialien oder für in der medizinischen Prothetik.

Als auto- oder kaltpolymerisierbar gilt ein Prothesenbasismaterial gemäss der Erfindung, wenn die Kriterien nach ISO 20795-1 (Pkt. 3.1) erfüllt sind. Als kaltpolymerisierende Kunststoffe gelten Zusammensetzungen, die unterhalb 65 °C polymerisieren. Erfindungsgemässe kaltpolymerisierende Prothesenbasismaterialien können vorzugsweise in einem Temperaturbereich nach Mischen der beiden Komponenten (A) und (B) von 50 °C bis 65 °C, vorzugsweise von 50 bis 60 °C, weiter bevorzugt von 50 °C bis 55 °C selbständig aushärten bzw. auspolymerisieren. Vorzugsweise erfolgt die Polymerisation über 5 bis 180 Minuten, bevorzugt für 30 bis 60 Minuten, besonders bevorzugt für 30 Minuten. Entsprechend der vorstehenden Norm werden polymerisierbare Zusammensetzungen, die ab 65 °C selbständig aushärten bzw. auspolymerisieren, als heisshärtende Zusammensetzungen, bezeichnet.

Die Pulverkomponente des Zweikomponenten-Prothesenbasismaterials enthält in der Regel als pulverförmige Komponente einen polymeren Partikel, insbesondere auf Methacrylatbasis, und/oder ein Perlpolymerisat auf Methacrylat-Basis. Perlpolymerisate werden auf diesem Gebiet häufig als Pulver bezeichnet.

In einer bevorzugten Ausgestaltung sind in die Perlen des ersten (Co)polymerisats und/oder in die Perlen des zweiten (Co)polymerisats zumindest teilweise Vernetzer einpolymerisiert worden. Die ersten und zweiten Perlpolymerisate umfassen somit auch vernetzte und teilvernetzte Perlpolymerisate.

Für die Vernetzung greift man regelmässig auf multifunktionelle Comonomere oder auch multifunktionelle Oligomere zurück. Neben di-, tri- und polyfunktionellen (Meth)acrylaten eignen sich hierfür auch Pfropfvernetzer mit mindestens zwei unterschiedlichen reaktiven C-C-Doppelbindungen, beispielsweise Alkylmethacrylaten und Alkylacrylaten, sowie aromatische Vernetzer wie 1,2-Divinylbenzol, 1,3-Divinylbenzol und 1,4-Divinylbenzol. Unter den difunktionellen (Meth)acrylaten seien insbesondere die (Meth)acrylate des Propandiols, Butandiols, Hexandiols, Octandiols, Nonandiols, Decandiols und Eicosandiols sowie ferner die Di(meth)acrylate des Ethylenglycols, Triethylenglycols, Tetraethylenglycols, Dodecaethylenglycols, Tetradecaethylenglycols, Propylenglycols, Dipropylenglycols und Tetradecapropylenglycols, ausserdem Glycerindi(meth)acrylat, 2,2-bis[(gamma-methacryloxy-beta-oxypropoxy)-phenylpropan], Neopentylglycol-di(meth)acrylat, 2,2-di-methacryloxypoly-ethoxyphenyl)propan mit 2 bis 10 Ethoxygruppen pro Molekül sowie 1,2-Bis(3-methacryloxy-2-hydroxypropoxy)butan genannt. Exemplarisch seien als multifunktionelle (Meth)acrylate, z. B. Di-, Tri- und/oder Tetra(meth)acrylate, wie 1,4-Butandioldimethacrylat, Ethylenglycoldimethacrylat sowie di-oder trivinylische Verbindungen wie Divinylbenzol hervorgehoben. Der Gehalt an derartigen Vernetzermolekülen liegt vorzugsweise im Bereich von 0,1 Gew.-% bis 10 Gew.-%, insbesondere im Bereich von 0,5 Gew.-% bis 5 Gew.-%, in der Ausgangsmischung für das Perlpoymerisat.

Das für die Polymerisation erforderliche radikalische Initiatorsystem ist, je nach Reaktionsbedingungen bzw. Polymerisationssystem, in der flüssigen Komponente (A) und/oder der pulverförmigen Komponente (B) enthalten. Diesbezügliche Details sind dem Fachmann bekannt. Beispielsweise liegt bei Basismischungen für Kaltpolymerisate das Initiatorsystem meist in beiden Komponenten, der flüssigen Komponente und der pulverförmigen Komponente vor und wird demgemäss beim Mischen dieser Komponenten zusammengeführt. Folglich liegt in der Regel eine Initiatorkomponente (c) in der pulverförmigen Komponente (B) vor, insbesondere in Form von (i) Barbitursäure oder Thiobarbitursäure oder ein Barbitursäure- oder Thiobarbitursäurederivaten. Ein weiterer Teil des Initiatorsystems (c) kann sich in der Flüssigkomponente (A) befinden, in der Regel ein Coinitiator. Bevorzugte Komponenten des Initiatorsystems umfassen (ii) mindestens ein Kupfersalz oder Kupferkomplex und (iii) mindestens eine Verbindung mit einem ionischen Halogenatom, besonders bevorzugt ist ein Redoxsystem umfassend 1-Benzyl-5-Phenylbarbitursäure, Kupferacetylacetonat, Triazin-Derivat, Toluidin-Derivat und/oder Benzyldibutylammoniumchlorid. Als radikalische Initiatorsysteme sind die genannten Redoxsysteme geeignet. In einer zweckmässigen Ausführungsform enthält ein solches Redoxsystem Barbitursäure oder Thiobarbitursäure oder ein Barbitursäure- oder Thiobarbitursäurederivat (beispielsweise 25 bis 80 Gew.-%), mindestens ein Kupfersalz oder ein Kupferkomplex (beispielsweise 0,1 bis 8 Gew.-%) und mindestens eine Verbindung mit einem ionogen vorliegenden Halogenatom (beispielsweise 0,05 bis 7 Gew.-%). Exemplarisch seien als geeignete Bestandteile des vorangehend genannten Redoxsystems 1-Benzyl-5-Phenylbarbitursäure, Kupferacetylacetonat, Kupfer-II-Chlorid, Triazin-Derivat, Toluidin-Derivat und/oder Benzyldibutylammoniumchlorid genannt.

Vorzugsweise umfasst der mindestens eine Initiator oder die mindestens eine Komponente des Initiatorsystem für die Autopolymerisation mindestens ein Initiatorsystem ausgewählt aus Redoxsystemen, umfassend ein Oxidationsmittel und ein Reduktionsmittel ausgewählt aus Barbitursäure oder ein Barbitursäurederivat, Sulfinsäure, Sulfinsäurederviat oder Mischungen umfassend mindestens zwei der vorgenannten Reduktionsmittel, besonders bevorzugt ist ein Redoxsystem umfassend (i) mindestens eine Barbitursäure oder Thiobarbitursäure oder ein Barbitursäure- oder Thiobarbitursäurederivat oder Mischungen umfassend mindestens zwei der vorgenannten Reduktionsmittel, vorzugsweise in der pulverförmigen Komponente, und (ii) mindestens ein Kupfersalz, wie Kupfer-II-chlorid, oder Kupferkomplex und (iii) mindestens eine Verbindung mit einem ionischen Halogenatom, insbesondere liegen (ii) und (iii) in der flüssigen Monomerkomponente vor, besonders bevorzugt ist ein Redoxsystem umfassend 1-Benzyl-5-Phenylbarbitursäure, Kupferacetylacetonat und Triazin-Derivat, Toluidin-Derivat und/oder Benzyldibutylammoniumchlorid. Das Toluidin-Derivat wird als Co-Initiator für die Barbitursäure eingesetzt. Optional kann das System (iv) mindestens ein Peroxid umfassen. Dabei liegen die Komponenten (ii) und (iii) vorzugsweise in der flüssigen Monomerkomponente A vor und die Komponenten (i) und optional (iv) vorzugsweise in der pulverförmigen Komponenten vor.

Die Aushärtung der Zusammensetzungen erfolgt vorzugsweise durch redoxinduzierte radikalische Polymerisation bei Raumtemperatur bzw. bei leicht erhöhter Temperatur unter leichtem Druck, um Blasenbildung zu vermeiden. Ein besonders bevorzugtes Initiatorsystem ist eine Kombination von Barbitursäuren in Verbindung mit Kupfer- und Chloridionen. Dieses System zeichnet sich im Unterschied zu Peroxid-Aminsystemen durch eine hohe Farbstabilität und geringere Toxizität aus.

Ferner kann man die pulverförmige Komponente (B) und/oder die Flüssigkomponente (A) in bekannter Weise mit weiteren Zusatzstoffen aus den Gruppen der Stabilisatoren, UV-Absorbern, Thixotropiermitteln und Füllstoffen versehen.

Die Erfindung wird durch die nachgeltenden Bespiele näher erläutert, ohne die Erfindung auf diese Ausführungsbeispiele zu beschränken.

### Ausführungsbeispiele:

Herstellung einer Pulvermischung: Aus drei PMMA-basierten Perlen wird zusammen mit Barbitursäure ein trimodales Pulversystem durch Mischen hergestellt. Die Herstellung der Flüssigkeit erfolgte aus den nachfolgend angegebenen Komponenten durch Mischen.

Die nachfolgenden Messwerte wurden entsprechend der Norm DIN EN ISO 20795-1 bestimmt. Der Rest-MMA-Gehalt wurde ebenfalls entsprechend dieser Norm analog Punkt 8.8 ermittelt. Punkt 5.2.10 definiert, dass für Materialien mit erhöhter Schlagzähigkeit der Höchstfaktor der Beanspruchungsintensität mindestens 1,9 MPa m^{1/2} betragen muss. Die Bestimmung erfolgt gemäß Punkt 8.6. Punkt 5.2.11 definiert, dass die Gesamtbrucharbeit mindestens 900 J/m² betragen muss. Die Messung der Bruchzähigkeit wurde analog der Norm EN ISO 20795-1:2013 gemäß Punkt 8.6 durchgeführt. Das Gerät hieß: Zwick/Roell Z010, Maschinentyp TMT1-FR010TN.A50. In den Beispielen wurde als Fraktion mit dem grössten Partikeldurchmesser eine Core-Shell-Perle eingesetzt, wie die 6681F, wobei auch normale Perlen eingesetzt werden können. Beispiele 1-3: Die Kunststoffe wurden alle im Verhältnis 10:7 und mittels Gießverfahren hergestellt. Beispiel 4 wurde im Verhältnis 10 : 5 hergestellt. Die Bestimmung erfolgte gemäß Punkt 8.6. Die Prüfkörper wurden für 30 min bei 55 °C und 2 bar Druck polymerisiert.

### Beispiel 1

| **Flüssigkeit** | **Gew.-%** |
|---|---|
| MMA | 83,435 |
| Aliquat 2-(4,6-diphenyl-1,3,5-triazin-2-yl)-5-((hexyl)oxy)-phenol | 0,15 |
| Kupfer(II) Chlorid Lsg. | 0,07 |
| UV-Stabilisator | 0,25 |
| N,N-Bis(2(hydroxyethyl)-p-toluidin | 0,1 |
| n-butyl acryloxyethyl-Carbamat | 10 |
| difunktionelles, aliphatisches Urethanacrylat-Oligomer | 3 |
| Tris-(2-Hydroxyethyl)-isocynaurat-Triacrylat | 1 |
| Styrol-Butylacrylat-Core Shell Partikel (200 bis 400 nm) | 2 |
| | |

| **Pulver** | **Gew.-%** |
|---|---|
| Perle 1 d₅₀: 35 µm | 77,56 |
| Perle 2 d₅₀: 45 µm | 5 |
| Perle 3 d₅₀ 60 µm | 15 |
| Barbitursäure | 2,44 |

Entsprechend der üblichen Verarbeitungsweise für Prothesenbasismaterialien werden Prüfkörper gemäss der Norm DIN EN ISO 20795-1 (39 mm x 8 mm x 4 mm) zur Bestimmung der physikalischen Eigenschaften im Verhältnis Pulver zu Flüssigkeit von 10:7 hergestellt. Folgende Messwerte wurden bestimmt:

| | Bsp. 1 |
|---|---|
| Mechanik 10:7 - Biegefestigkeit [Mpa] | 70,8 |
| Mechanik 10:7 I - E-Modul [Mpa] | 2171 |
| Mechanik 10:7 - Bruchzähigkeit als Höchstfaktor der Beanspruchungsintensität Kmax [Mpa m^{1/2}] | 2,54 |
| Mechanik 10:7 I - Bruchzähigkeit als Gesamtbrucharbeit Wf [J/m²] | 1298 |
| Maximale Temperatur 10:7 [°C] | 72 °C* |

| | |
|---|---|
| * gemessen bei RT gestarteter Polymerisation. | |

Farbprüfkörper die in Metallformen hergestellt wurden, zeigen eine Transparenz von 96 %. Der Rest-MMA-Gehalt 48 Stunden nach Herstellung (Normprüfung) beträgt bei einer Polymerisationszeit von 30 min 2,0, bei 60 min 1,8 Gew.-%. Bei einer Polymerisationszeit von 30 min beträgt der Restmonomergehalt an MMA 4 Tage nach der Herstellung 1,8 Gew.-%.

### Beispiel 2: Analog Beispiel 1 werden Prüfkörper entsprechend der in der Tabelle angegebenen Zusammensetzung hergestellt.

| **Flüssigkeit** | **Gew.-%** |
|---|---|
| MMA | 88,43 |
| Aliquat 2-(4,6-diphenyl-1,3,5-triazin-2-yl)-5-((hexyl)oxy)-phenol | 0,15 |
| Kupfer(II) Chlorid Lsg. | 0,07 |
| UV-Stabilisator | 0,25 |
| N,N-Bis(2(hydroxyethyl)-p-toluidin | 0,1 |
| n-butyl acryloxyethyl-Carbamat | 5 |
| difunktionelles, aliphatisches Urethanacrylat-Olygomer | 3 |
| Tris-(2-Hydroxyethyl)-isocynaurat-Triacrylat | 1 |
| Styrol-Butylacrylat-Core Shell Partikel | 2 |
| ges. | 100 |
| | |
| | |

| **Pulver** | **Gew.-%** |
|---|---|
| Perle 1 d₅₀: 45 µm | 67,56 |
| Perle 2 d₅₀: 45 µm | 15 |
| Perle 3 d₅₀ 60 µm | 15 |
| Barbitursäure | 2,44 |

Folgende Messwerte wurden nach der vorstehenden Norm (DIN EN ISO 20795-1) bestimmt:

| | |
|---|---|
| Mechanik 10:7 - Biegefestigkeit [Mpa] | 68,1 |
| Mechanik 10:7 I - E-Modul [Mpa] | 2307 |
| Mechanik 10:7 - Bruchzähigkeit als Höchstfaktor der Beanspruchungsintensität Kmax [Mpa m^{1/2}] | 2,37 |
| Mechanik 10:7 I - Bruchzähigkeit als Gesamtbrucharbeit Wf [J/m²] | 952,7 |
| Maximale Temperatur 10:7 [°C] | 110,5* |

| | |
|---|---|
| * gemessen bei RT gestarteter Polymerisation. | |

Die Prüfkörper zeigen eine niedrige Transparenz, da die PMMA-Perlen noch sichtbar sind. Die Verarbeitungszeit ist deutlich zu kurz. Zwar können High-Impact-Eigenschaften erreicht werden, aber die ungeeignete Abmischung der Perlen führt zu einem ungünstigen Temperaturprofil, indem die Polymerisationstemperaturen zu hoch ansteigen können und damit zu Einbußen in Verarbeitung und Transparenz führen.

### Beispiel 3: Analog Beispiel 1 werden Prüfkörper entsprechend der in der Tabelle angegebenen Zusammensetzung hergestellt.

| **Flüssigkeit** | **Gew.-%** |
|---|---|
| MMA | 88,43 |
| Aliquat 2-(4,6-diphenyl-1,3,5-triazin-2-yl)-5-((hexyl)oxy)-phenol | 0,15 |
| Kupfer(II) Chlorid Lsg. | 0,07 |
| UV-Stabilisator | 0,25 |
| N,N-Bis(2(hydroxyethyl)-p-toluidin | 0,1 |
| n-butyl acryloxyethyl-Carbamat | 20 |
| difunktionelles, aliphatisches Urethanacrylat-Olygomer | 3 |
| Tris-(2-Hydroxyethyl)-isocynaurat-Triacrylat | 1 |
| Styrol-Butylacrylat-Core Shell Partikel | 2 |
| ges. 100 | |

| **Pulver** | **Gew.-%** |
|---|---|
| Perle 1 d₅₀: 45 µm | 67,56 |
| Perle 2 d₅₀: 45 µm | 15 |
| Perle 3 d₅₀ 60 µm | 15 |
| Barbitursäure | 2,44 |

Folgende Messwerte wurden bestimmt:

| | |
|---|---|
| Mechanik 10:7 - Biegefestigkeit [Mpa] | 61,0 |
| Mechanik 10:7 I - E-Modul [Mpa] | 2159 |
| Mechanik 10:7 - Bruchzähigkeit als Höchstfaktor der Beanspruchungsintensität Kₘₐₓ [Mpa m^{1/2}] | 1,92 |
| Mechanik 10:7 I - Bruchzähigkeit als Gesamtbrucharbeit Wf [J/m²] | 653,8 |
| Maximale Temperatur 10:7 [°C] | 66,7 °C* |

| | |
|---|---|
| * gemessen bei RT gestarteter Polymerisation. | |

Die Proben enthalten einen Rest-MMA-Gehalt von 0,9 Gew.-%. Allerdings werden die High-Impact-Eigenschaften nach Norm nicht erreicht aufgrund nicht idealer Verhältnisse in der Monomermatrix und Perlmischung.

### Beispiel 4: Die pulverförmige Komponente sowie die flüssige Komponente wurden im Verhältnis 10:7 gemischt, so dass sich die folgende Zusammensetzung ergibt.

| **(Verhältnis 10:7)** | Gew.-% |
|---|---|
| MMA | Ca. 34 |
| Aliquat 2-(4,6-diphenyl-1,3,5-triazin-2-yl)-5-((hexyl)oxy)-phenol | < 1 |
| Kupfer(II) Chlorid Lsg. | < 1 |
| UV-Stabilisator | < 1 |
| N,N-Bis(2(hydroxyethyl)-p-toluidin | < 1 |
| n-butyl acryloxyethyl-Carbamat | 4,2 |
| difunktionelles, aliphatisches Urethanacrylat-Olygomer | < 2 |
| Tris (2-hydroxy ethyl) Isocyanurat- triacrylat | < 1 |
| Styrol-Butylacrylat-Core Shell Partikel | < 1 |
| Perle 1 d50: 35 µm | Ca. 46 |
| Perle 2 d50: 45 µm | 3 |
| Perle 3 d50 60 µm | 9 |
| Barbitursäure | < 2 |
| | auf 100 Gew.-% |

### Beispiel 5: Die pulverförmige Komponente sowie die flüssige Komponente wurden nachfolgend im Verhältnis 10:5 gemischt, so dass sich die folgende Zusammensetzung ergibt.

| **(Verhältnis 10:5)** | **Gew.-%** |
|---|---|
| MMA | Ca. 28 |
| Aliquat 2-(4,6-diphenyl-1,3,5-triazin-2-yl)-5-((hexyl)oxy)-phenol | 0,5 |
| Kupfer(II) Chlorid Lsg. | < 1 |
| UV-Stabilisator | < 1 |
| N,N-Bis(2(hydroxyethyl)-p-toluidin | < 1 |
| n-butyl acryloxyethyl-Carbamat | 3,3 |
| difunktionelles, aliphatisches Urethanacrylat-Olygomer | < 1 |
| Tris (2-hydroxy ethyl) Isocyanurat-triacrylat | < 1 |
| Styrol-Butylacrylat-Core Shell Partikel | < 1 |
| Perle 1 d₅₀: 35 µm | 52 |
| Perle 2 d₅₀: 45 µm | 3,3 |
| Perle 3 d₅₀ 60 µm | 10 |
| Barbitursäure | 1,6 |
| | auf 100 Gew.-% |

Das auspolymerisierte Prothesenbasismaterial weist eine Gesamtbrucharbeit von 1126,89 J/m² und einen Höchstfaktor der Beanspruchungsintensität von 2,53 MPa m^{1/2} auf. Das E-Modul beträgt 2273 kJ/mol. Um Prothesen mit optimaler Passung zu erzeugen, wird der im Verhältnis Pulver zu Flüssigkeit von 10:5 angemischte Teig mittels dem Injektionsgerät Palajet (in eine Gipsform) injiziert. Auch mit dieser Applikationsmethode werden High Impact-Werte erzielt.

Die aus der erfindungsgemässen Monomermischung hergestellten Prothesenbasismaterialien zeigen eine deutlich verbesserte Bruchzähigkeit gegenüber allen Vergleichsbeispielen, eine erhöhte Transparenz sowie den geringsten Restmonomergehalt an MMA.

**Farbprüfkörper:** Folgende Pulver- und Monomermischungen werden im Verhältnis 10 g Pulver zu: 7 ml Flüssigkeit intensiv gemischt und nach der Anquellphase (ca. 5 min bei 23 °C) Prüfkörper mit einer Abmessung von 30 x 30 x 3 mm in einer Metallform gegossen und 30 min. bei 55 °C und 2 bar Druck im Palamat elite auspolymerisiert. Die Transparenzmessungen wurden mit dem Farbmessgerät FS600 Datacolor durchgeführt.

**Prüfkörper für mechanische Festigkeit:** Folgende Pulver- und Monomermischungen werden im Verhältnis 10 g Pulver: 7 ml Flüssigkeit intensiv gemischt und nach der Anquellphase (ca. 5 min bei 23 °C) Prüfkörper mit einer Abmessung von 100 x 100 x 5 mm gegossen und 30 min. bei 55 °C und 2 bar Druck im Palamat elite auspolymerisiert. Die Prüfplatten werden anschliessend auf die in der ISO 20795-1 angegebene Geometrie zurecht gesägt und geschliffen. Die Prüfkörper für mechanische du farbmetrischen Abprüfungen werden in Stahlformen hergestellt.

## Patentansprüche

1. Autopolymerisierbares 2-Komponenten-Prothesenbasismaterial umfassend
A) mindestens eine flüssige Monomerkomponente,
B) mindestens eine pulverförmige Komponente,
**dadurch gekennzeichnet, dass**
die Komponente (A) umfasst
(i) mindestens Methyl(meth)acrylat,
(ii) mindestens ein N-Alkyl- oder N-Alkenyl-substituiertes Acryloyloxy-Carbamat mit einer Molmasse von kleiner gleich 250 g/mol,
(iii) optional mindestens ein mindestens difunktionelles Urethan(meth)acrylat,
(iv) mindestens ein di-, tri-, tetra- oder multi-funktionelles Monomer, das kein Urethan(meth)acrylat ist,
(v) optional polymere Partikel mit einer Primärpartikelgröße von kleiner 800 nm,
(vi) mindestens einen Initiator oder mindestens eine Komponente eines Initiatorsystems für die Autopolymerisation,
und Komponente (B) umfasst
(i) mindestens eine pulverförmige Komponente polymerer Partikel, die mindestens drei verschiedene Fraktionen von Partikelgrößen polymerer Partikel umfasst, sowie
(ii) mindestens einen Initiator oder mindestens eine Komponente eines Initiatorsystems für die Autopolymerisation.

2. Prothesenbasismaterial nach Anspruch 1, **dadurch gekennzeichnet, dass** jeweils unabhängig die Komponente (A) umfasst
(v) polymere Partikel, die als durch eine elastischen Phase modifizierte Kern-Schale-Partikel vorliegen, und/oder unabhängig umfasst die Komponente (B) mindestens eine Fraktion (i) polymerer Partikel, die als durch eine elastische Phase modifizierte Kern-Schale Partikel vorliegen.

3. Prothesenbasismaterial nach Anspruch 1, **dadurch gekennzeichnet, dass** (i) die mittlere Partikelgröße jeder Faktion der mindestens drei Fraktionen von Partikelgrößen um mindestens 5 Mikrometer von der mittleren Partikelgrößen der beiden anderen Fraktionen beabstandet ist, und insbesondere die mittleren Partikelgröße aller Fraktionen im Bereich von 10 bis 120 Mikrometer liegt.

4. Prothesenbasismaterial nach einem der Ansprüche 1 bis 3, **dadurch gekennzeichnet, dass** die Komponente (B) als pulverförmige Komponente polymere Partikel mit drei Fraktionen umfasst, die ausgewählt sind
1) aus polymeren Partikeln einer mittleren Partikelgröße von
a) von 25 bis kleiner 40 µm,
b) von 40 bis kleiner 55 µm,
c) von 55 bis 100 µm, oder
2) aus polymeren Partikeln einer mittleren Partikelgröße von
a) von 35 µm mit plus/minus 2,5 µm
b) von 45 µm mit plus/minus 2,5 µm
c) von 60 µm mit plus/minus 2,5 µm,
wobei das Gewichtsverhältnis von a) zu b) zu c) von 12 bis 18 : 1 : 1 bis 5 beträgt.

5. Prothesenbasismaterial nach einem der Ansprüche 1 bis 4, **dadurch gekennzeichnet, dass** (ii) das N-Alkyl- oder N-Alkenyl-substituierte Acryloyloxy-Carbamat ein N-Alkyl- oder N-Alkenyl-substituiertes Acryloyloxy-Alkylen-Carbamat ist.

6. Prothesenbasismaterial nach einem der Ansprüche 1 bis 5, **dadurch gekennzeichnet, dass** (ii) das N-Alkyl substituierte Acryloyloxy-Carbamat n-Butyl Acryloyloxy-ethyl-carbamate ist (BAEC).

7. Prothesenbasismaterial nach einem der Ansprüche 1 bis 6, **dadurch gekennzeichnet, dass** der mindestens eine Initiator oder die mindestens eine Komponente des Initiatorsystems für die Autopolymerisation umfasst mindestens ein Initiatorsystem ausgewählt aus Redoxsystemen, umfassend Oxidationsmittel und ein Reduktionsmittel ausgewählt aus Ascorbinsäure, Ascorbinsäurederivat, Barbitursäure oder ein Barbitursäurederivat, Sulfinsäure, Sulfinsäurederviat, besonders bevorzugt ist ein Redoxsystem umfassend (i) Barbitursäure oder Thiobarbitursäure oder ein Barbitursäure- oder Thiobarbitursäurederivat und (ii) mindestens ein Kupfersalz oder Kupferkomplex und (iii) mindestens eine Verbindung mit einem ionischen Halogenatom, besonders bevorzugt ist ein Redoxsystem umfassend 1-Benzyl-5-Phenylbarbitursäure, Kupferacetylacetonat und ein Triazin-Derivat, Toluidin-Derivat und/oder Benzyldibutylammoniumchlorid.

8. Prothesenbasismaterial nach einem der Ansprüche 1 bis 7, **dadurch gekennzeichnet, dass** das Prothesenbasismaterial umfassend die Komponente (A) und (B) umfasst
(i) 20 bis 50 Gew.-% Methylmethacrylat sowie optional mindestens ein (2-Alkyl)-Acrylsäureester, der nicht Methylmethacrylat ist,
(ii) 1 bis 30 Gew.-% mindestens ein N-Alkyl- oder N-Alkenyl-substituiertes Acryloyloxy-Carbamat mit einer Molmasse von kleiner gleich 250 g/mol,
(iii) 0,5 bis 10 Gew.-% mindestens ein mindestens difunktionelles Urethan(meth)acrylat,
(iv) 0,05 bis 10 Gew.-% mindestens ein di-, tri-, tetra- oder multi-funktionelles Monomer, das kein Urethan(meth)acrylat ist,
(v) 0,1 bis 10 Gew.-% polymere Partikel, die als durch eine elastischen Phase modifizierte Kern-Schale-Partikel vorliegen, mit einer Primärpartikelgröße von kleiner 800 nm,
(vi) 0,05 bis 2 Gew.-% mindestens einen Initiator oder mindestens eine Komponente eines Initiatorsystems für die Autopolymerisation, sowie
(vii) 48,3 bis 78,3 Gew.-%, insbesondere 55 bis 65 Gew.-%, mindestens eine pulverförmige Komponente polymerer Partikel, die mindestens drei verschiedene Fraktionen von Partikelgrößen polymerer Partikel umfasst, wobei sich die vorstehenden Angaben in Gew.-% auf die Gesamtzusammensetzung von (A) und (B) beziehen, wobei die drei Fraktionen ausgewählt sind aus polymeren Partikeln einer mittleren Partikelgröße
a) von 25 bis kleiner 40 µm, die zu 50 bis 90 Gew.-%,
b) von 40 bis kleiner 55 µm, die zu 0,1 bis 20 Gew.-%, und
c) von 55 bis 100 µm, die zu 0,5 bis 30 Gew.-% vorliegen, wobei sich die Angaben in Gew.-% von a), b) und c) auf die pulverförmige Komponente (B) beziehen.

9. Verfahren zur Herstellung eines auspolymerisierten Prothesenbasismaterials, indem die Komponenten
A) mindestens eine flüssige Monomerkomponente, und
B) mindestens eine pulverförmige Komponente, des Prothesenbasismaterials nach einem der Ansprüche 1 bis 8, gemischt werden und nachfolgend auspolymerisiert werden.

10. Verfahren nach Anspruch 9, **dadurch gekennzeichnet, dass** die A) Monomerkomponente und die B) pulverförmige Komponente im Gewichtsverhältnis von 1 : 10 bis 10 : 1, insbesondere im Gewichtsverhältnis 8 bis 12 der pulverförmigen Komponente zu 4 bis 8 der Monomerkomponente gemischt werden.

11. Auspolymerisiertes Prothesenbasismaterial erhältlich nach einem Verfahren nach einem der Ansprüche 9 oder 10.

12. Auspolymerisiertes Prothesenbasismaterial nach Anspruch 11, **dadurch gekennzeichnet, dass** es einen Restmonomergehalt an Methylmethacrylat von kleiner gleich 3 Gew.-% bestimmt nach ISO 20795-1:2013 aufweist, insbesondere weist es einen Restmonomergehalt an Methylmethacrylat von kleiner gleich 2,5 Gew.-% auf, bevorzugt von kleiner gleich 2,4, 2,3, 2,2, 2,1, 2,05 Gew.-%, insbesondere mit einer Schwankungsbreite von +/- 0,05 Gew.-%.

13. Auspolymerisiertes Prothesenbasismaterial nach Anspruch 11 oder 12, **dadurch gekennzeichnet, dass** das Prothesenbasismaterial eine Transparenz von größer gleich 95 % (gemessen an in Metallformen hergestellten Farbprüfkörpern der Dicke 3 mm) aufweist.

14. Verwendung des Prothesenbasismaterials nach einem der vorstehenden Ansprüche im humanen Dentalbereich zur Herstellung von Dentalprothesen, Teilen von Prothesen, zur Anfertigung von Aufbissschienen, von Bohrschablonen für die Implantologie, von Mouthguards, zur Herstellung eines Fräsrohlings, Gelenkprothese, Krone, Teleskops, Veneers, Zahnbrücke, Prothesenzahns, Implantateils, Abutments, Suprastruktur, kieferorthopädischen Apparats und Instruments, prothetischer Formkörper in Form von Knochen oder Teilen davon, prothetischer Formkörper im Veterinärbereich, zur Herstellung eines Hufteils oder von Hufreparaturmaterialien, zur Herstellung von Knochenzement, zur Herstellung von Knochenzement zur Zementierung von künstlichen Gelenkprothesen, Kronen, Teleskopen, Veneers, Zahnbrücken, Prothesenzähnen, Implantaten, Implantatteilen, Abutments, Suprastrukturen.

15. Kit umfassend ein autopolymerisierbares Prothesenbasismaterial, wobei das Kit separierte Komponenten (A) und (B) umfasst, **dadurch gekennzeichnet, dass** die Komponente A umfasst
(i) 60 bis 85 Gew.-% Methylmethacrylat,
(ii) 5 bis 20 Gew.-% mindestens ein N-Alkyl- oder N-Alkenyl-substituiertes Acryloyloxy-Carbamat mit einer Molmasse von kleiner gleich 250 g/mol,
(iii) 0,5 bis 10 Gew.-% mindestens ein mindestens difunktionelles Urethan(meth)acrylat,
(iv) 0,05 bis 10 Gew.-% mindestens ein di-, tri-, tetra- oder multi-funktionelles Monomer, das kein Urethan(meth)acrylat ist
(v) 0,1 bis 10 Gew.-% polymere Partikel, die als durch eine elastischen Phase modifizierte Kern-Schale-Partikel vorliegen, mit einer Primärpartikelgröße von kleiner 800 nm,
(vi) 0,05 bis 2 Gew.-% mindestens einen Initiator oder mindestens eine Komponente eines Initiatorsystems für die Autopolymerisation, wobei sich die vorstehenden Angaben in Gew.-% auf die Gesamtzusammensetzung der Komponente (A) beziehen, sowie
als Komponente (B) umfasst
(i) 90 bis 99,95 Gew.-% mindestens eine pulverförmige Komponente polymerer Partikel, die mindestens drei verschiedene Fraktionen von Partikelgrößen polymerer Partikel umfasst, wobei die drei Fraktionen ausgewählt sind aus polymeren Partikeln einer mittleren Partikelgröße
a) von 25 bis kleiner 40 µm, die zu 50 bis 90 Gew..-%,
b) von 40 bis kleiner 55 µm, die zu 0,1 bis 20 Gew.-%, und
c) von 55 bis 100 µm, die zu 0,5 bis 30 Gew.-% vorliegen, und
(ii) 0,05 bis 10 Gew.-% mindestens einen Initiator oder mindestens eine Komponente eines Initiatorsystems für die Autopolymerisation, wobei sich die vorstehenden Angaben in Gew.-% auf die Gesamtzusammensetzung der Komponente (B) beziehen.

## Claims

1. Autopolymerisable 2-component prosthetic base material comprising
A) at least one liquid monomer component,
B) at least one powdered component,
**characterised in that**
component (A) comprises
(i) at least methyl (meth)acrylate,
(ii) at least one N-alkyl or N-alkenyl substituted acryloyloxy carbamate having a molecular mass of less than or equal to 250 g/mol,
(iii) optionally, at least one at least di-functional urethane (meth)acrylate,
(iv) at least one di-, tri, tetra- or multi-functional monomer not being urethane (meth)acrylate,
(v) optionally, polymeric particles having a primary particle size of less than 800 nm,
(vi) at least one initiator or at least one component of an initiator system for autopolymerisation,
and component (B) comprises
(i) at least one powdered component of polymeric particles comprising at least three different fractions of particle sizes of polymeric particles, as well as
(ii) at least one initiator or at least one component of an initiator system for autopolymerisation.

2. Prosthetic base material according to claim 1, **characterised in that** component (A) respectively independently comprises
(v) polymeric particles being present as core-shell particles modified by an elastic phase, and/or independently component (B) comprises at least one fraction (i) of polymeric particles being present as core-shell particles modified by an elastic phase.

3. Prosthetic base material according to claim 1, **characterised in that** (i) the average particle size of each fraction of the at least three fractions of particle sizes is at least 5 micrometers apart from the average particle size of the other two fractions, and in particular the average particle sizes of all fractions are in the range of 10 to 120 micrometers.

4. Prosthetic base material according to any one of the claims 1 to 3, **characterised in that** component (B) comprises as powdered component polymeric particles with three fractions, which are selected
1) from polymeric particles of an average particle size
a) of 25 to less than 40 µm,
b) of 40 to less than 55 µm,
c) of 55 to 100 µm, or
2) from polymeric particles of an average particle size
a) of 35 µm with plus/minus 2.5 µm
b) of 45 µm with plus/minus 2.5 µm
c) of 60 µm with plus/minus 2.5 µm,
the weight ration of a) to b) to c) being from 12 to 18 : 1 : 1 to 5.

5. Prosthetic base material according to any one of the claims 1 to 4, **characterised in that** (ii) the N-alkyl or N-alkenyl substituted acryloyloxy carbamate is an N-alkyl or N-alkenyl substituted acryloyloxy alkylene carbamate.

6. Prosthetic base material according to any one of the claims 1 to 5, **characterised in that** (ii) the N-alkyl substituted acryloyloxy carbamate is n-butyl acryloyloxy ethyl carbamate (BAEC).

7. Prosthetic base material according to any one of the claims 1 to 6, **characterised in that** the at least one initiator or the at least one component of an initiator system for autopolymerisation comprises at least one initiator system selected from redox system, comprising oxidising agent and a reducing agent selected from ascorbic acid, ascorbic acid derivative, barbituric acid or a barbituric acid derivative, sulfinic acid, sulfinic acid derivative, particularly preferred is a redox system comprising (i) barbituric acid or thiobarbituric acid or a barbituric acid derivative or thiobarbituric acid derivative, and (ii) at least one copper salt or one copper complex, and (iii) at least one compound having an ionic halogen atom, particularly preferred is a redox system comprising 1-benzyl-5-phenylbarbituric acid, copper acetylacetonate and a triazine derivative, toluidine derivative and/or benzyldibutylammonium chloride.

8. Prosthetic base material according to any one of the claims 1 to 7, **characterised in that** the prosthetic base material comprising components (A) and (B) comprises
(i) 20 to 50 % by weight methyl methacrylate, as well as, optionally, at least one (2-alkyl) acrylic acid ester not being methyl methacrylate,
(ii) 1 to 30 % by weight at least one N-alkyl or N-alkenyl substituted acryloyloxy carbamate having a molecular mass of less than or equal to 250 g/mol,
(iii) 0.5 to 10 % by weight at least one at least di-functional urethane (meth)acrylate,
(iv) 0.05 to 10 % at least one di-, tri, tetra- or multi-functional monomer not being urethane (meth)acrylate,
(v) 0.1 to 10 % by weight polymeric particles being present as core-shell particles modified by an elastic phase, having a primary particle size of less than 800 nm,
(vi) 0.05 to 2 % by weight at least one initiator or at least one component of an initiator system for autopolymerisation, as well as
(vii) 48.3 to 78.3 % by weight, in particular 55 to 65 % by weight, at least one powdered component of polymeric particles comprising at least three different fractions of particle sizes of polymeric particles, the above information in % by weight being based on the total composition of (A) and (B), the three fractions being selected from polymeric particles of an average particle size
a) of 25 to less than 40 µm, being present at 50 to 90 % by weight,
b) of 40 to less than 55 µm, being present at 0.1 to 20 % by weight, and
c) of 55 to 100 µm, being present at 0.5 to 30 % by weight, the information in % by weight of a), b) and c) being based on powdered component (B).

9. Method for the production of a polymerised prosthetic base material, in which components
A) at least one liquid monomer component, and
B) at least one powdered component, of the
prosthetic base material according to any one of the claims 1 to 8 are being mixed and subsequently polymerised.

10. Method according to claim 9, **characterised in that** the
A) monomer component and the B) powdered component are being mixed at a weight ratio of 1 : 10 to 10 : 1, in particular at a weight ratio of 8 to 12 of the powdered component to 4 to 8 of the monomer component.

11. Polymerised prosthetic base material obtainable according to a method according to any one of the claims 9 or 10.

12. Polymerised prosthetic base material according to claim 11, **characterised in** comprising a residual monomer content of methylmethacrylate of less than or equal to 3 % by weight determined according to ISO 20795-1:2013, in particular it comprises a residual monomer content of methylmethacrylate of less than or equal to 2.5 % by weight, preferably of less than or equal to 2.4, 2.3, 2.2, 2.1, 2.05 % by weight, in particular having a range of variation of +/- 0.05 % by weight.

13. Polymerised prosthetic base material according to claim 11 or 12, **characterised in that** the prosthetic base material has a transparency of greater than or equal to 95 % (measured against colour specimens of thickness 3 mm produced in metal moulds).

14. Use of the prosthetic base material according to any one of the preceding claims in human dental field for producing dental protheses, parts of prostheses, for manufacture of occlusal splints, of surgical guides for implantology, of mouthguards, for producing a milling blank, joint prothesis, crown, telescopic prosthesis or telescopic crown, veneer, dental bridge, prosthetic tooth, implant part, abutment, superstructure, orthodontic appliances and instruments, prosthetic moulded bodies in the form of bones or parts thereof, prosthetic moulded bodies in veterinary filed, for producing a hoof part or hoof repair materials, for producing bone cement, for producing bone cement for cementing of artificial joint protheses, crowns, telescopic protheses or telescopic crowns, veneers, dental bridges, prosthetic teeth, implants, implant parts, abutments, superstructures.

15. Kit comprising an autopolymerisable prosthetic base material, the kit comprising separated components (A) and (B), **characterised in that** component (A) comprises
(i) 60 to 85 % by weight methylmethacrylate,
(ii) 5 to 20 % by weight at least one N-alkyl or N-alkenyl substituted acryloyloxy carbamate having a molecular mass of less than or equal to 250 g/mol,
(iii) 0.5 to 10 % by weight at least one at least di-function urethane (meth)acrylate,
(iv) 0.05 to 10 % by weight at least one di-, tri-, tetra- or multi-functional monomer not being urethane (meth)acrylate,
(v) 0.1 to 10 % by weight polymeric particles being present as core-shell particles modified by an elastic phase, having a primary particle size of less than 800 nm,
(vi) 0.05 to 2 % by weight at least one initiator or at least one component of an initiator system for autopolymerisation, the above information in % by weight being based on the total composition of component (A), as well as
component (B) comprises
(i) 90 to 99.95 % by weight at least one powdered component of polymeric particles comprising at least three fractions of particles sizes of polymeric particles, the three fractions being selected from polymeric particles of an average particle size
a) of 25 to less than 40 µm, being present at 50 to 90 % by weight,
b) of 40 to less than 55 µm, being present at 0.1 to 20 % by weight, and
c) of 55 to 100 µm, being present at 0.5 to 30 % by weight, and
(ii) 0.05 to 10 % by weight at least one initiator or at least one component of an initiator system for autopolymerisation, the above information in % by weight being based on the total composition of component (B).

## Revendications

1. Matériau de base prothétique à deux composantes autopolymérisable comprenant
A) au moins un composant monomère liquide,
B) au moins un composant pulvérulent,
**caractérisé en ce que**
le composant (A) comprend
(i) au moins (méth)acrylate de méthyle,
(ii) au moins un acryloyloxy carbamate N-alkyle-ou N-alkényle-substitué ayant une masse moléculaire d'inférieure ou égale à 250 g/mol,
(iii) facultativement, au moins un (méth)acrylate d'uréthane au moins di-fonctionnel,
(iv) au moins un monomère di-, tri-, tétra- ou multi-fonctionnel autre que le (méth)acrylate d'uréthane,
(v) facultativement, des particules polymériques ayant une taille de particules primaire d'inférieure à 800 nm,
(vi) au moins un initiateur ou au moins un composant d'un système d'initiateurs pour l'autopolymerisation,
et la composant (B) comprend
(i) au moins un composant pulvérulent des particules polymériques comprenant au moins trois fractions différentes des tailles de particules des particules polymériques,
(ii) au moins un initiateur ou au moins un composant d'un système d'initiateurs pour l'autopolymérisation.

2. Matériau de base prothétique selon la revendication 1, **caractérisé en ce que** le composant (A) comprend respectivement indépendamment
(v) des particules polymériques, étant présentes en tant que des particules cœur-coque modifiées par une phase élastique, et/ou indépendamment la composant (B) comprend au moins une fraction (i) des particules polymériques, étant présentes en tant que des particules cœur-coque modifiées par une phase élastique.

3. Matériau de base prothétique selon la revendication 1, **caractérisé en ce que** (i) la taille de particules moyenne de chaque fraction des au moins trois fractions des tailles de particules est au moins 5 micromètres espacée de la taille de particules moyenne des autres deux fractions, et en particulière la taille de particules moyenne de toutes les fractions est de l'ordre de 10 à 120 micromètres.

4. Matériau de base prothétique selon l'une des revendications 1 à 3, **caractérisé en ce que** le composant (B) en tant que le composant pulvérulent comprend des particules polymériques avec trois fractions, qui sont sélectionnées
1) parmi des particules polymériques d'une taille de particules
a) de 25 à inférieure à 40 µm,
b) de 40 à inférieure à 55 µm,
c) de 55 à 100 µm, ou
2) parmi des particules polymériques d'une taille de particules moyenne
a) de 35 µm avec plus/moins 2.5 µm
b) de 45 µm avec plus/moins 2.5 µm
c) de 60 µm avec plus/moins 2.5 µm,
le rapport en poids de a) à b) à c) étant de 12 à 18 : 1 : 1 à 5.

5. Matériau de base prothétique selon l'une des revendications 1 à 4, **caractérisé en ce que** (ii) l'acryloyloxy carbamate N-alkyl- ou N-alkényle-substitué est un acryloyloxy alkylene carbamate N-alkyl- ou N-alkényle-substitué.

6. Matériau des base prothétique selon l'une des revendications 1 à 5, **caractérisé en ce que** (ii) l'acryloyloxy carbamate N-alkyl-substitué est n-butyle acryloyloxy éthyle carbamate (BAEC).

7. Matériau de base prothétique selon l'une des revendications 1 à 6, **caractérisé en ce que** l'au moins un initiateur ou l'au moins composant du système d'initiateurs pour l'autopolymérisation comprend au moins un système d'initiateurs sélectionné parmi des systèmes redox, comprenant un agent oxydant et un agent réducteur sélectionné parmi de l'acide ascorbique, d'un dérivé d'acide ascorbique, de l'acide barbiturique ou d'un dérivé d'acide barbiturique, de l'acide sulfinique, d'un dérivé d'acide sulfinique, particulièrement préféré est un système redox comprenant (i) l'acide barbiturique ou l'acide thiobarbiturique ou un dérivé d'acide barbiturique ou d'acide thiobarbiturique, et (ii) au moins au moins un sel de cuivre ou un complexe de cuivre, et (iii) au moins un composé ayant un atome d'halogène ionique, particulièrement préféré est un système redox comprenant l'acide barbiturique de 1-benzyle-5-phényle, l'acétylacétonate de cuivre et un dérivé de triazine, un dérivé de toluidine et/ou le chlorure de benzyldibutylammonium.

8. Matériau de base prothétique selon l'une des revendications 1 à 7, **caractérisé en ce que** le de base matériau prothétique comprenant le composants (A) et (B) comprend
(i) 20 à 50 % en poids méthacrylate de méthyle, ainsi que, facultativement, au moins un ester d'acide acrylique (de 2-alkyl) autre que méthacrylate de méthyle,
(ii) 1 à 30 % en poids au moins un acryloyloxy carbamate N-alkyl ou N-alkényle-substitué ayant une masse moléculaire d'inférieure ou égale à 250 g/mol,
(iii) 0,5 à 10 % en poids au moins un (méth)acrylate d'uréthane au moins di-fonctionnel,
(iv) 0,05 à 10 % en poids au moins un monomère di-, tri-, tétra- ou multi-fonctionnel autre que le (méth)acrylate d'uréthane,
(v) 0,1 à 10 % en poids des particules polymériques, étant présentes en tant que des particules modifiées par une phase élastique ayant une taille de particules primaire d'inférieure à 800 nm
(vi) 0,05 à 2 % en poids au moins un initiateur ou au moins un composant d'un système d'initiateurs pour l'autopolymérisation, ainsi que
(vii) 48,3 à 78.3 % en poids, en particulière 55 à 65 % en poids, au moins un composant pulvérulent des particules polymériques comprenant au moins trois fractions différentes des tailles de particules des particules polymériques, les informations précédentes en % en poids étant basées sur la composition totale de (A) et (B), les trois fractions étant sélectionnées parmi des particules polymériques d'une taille de particules moyenne
a) de 25 à inférieure à 40 µm, étant présentes dans 50 à 90 % en poids,
b) de 40 à inférieure à 55 µm, étant présentes dans 0,1 à 20 % en poids, et
c) de 55 à 100 µm, étant présentes à 0,5 à 30 % en poids, les informations en % en poids de a), b) et c) étant basées sur le composant pulvérulent (B).

9. Procédé pour la production d'un matériau de base prothétique, dans lequel les composants
A) au moins un composant monomère liquide, et
B) au moins un composant pulvérulent, du matériau de base prothétique selon l'une des revendications 1 à 8 sont étant mélangés et ensuite polymérisés.

10. Procédé selon la revendication 9, **caractérisé en ce que** le
A) composant monomère et le B) composant pulvérulent sont été mélangés en rapport en poids de 1 : 10 à 10 : 1, en particulière en rapport en poids de 8 à 12 du composant pulvérulent à 4 à 8 du composant monomère.

11. Matériau de base prothétique polymérisé obtenable par un procédé selon l'une des revendications 9 ou 10.

12. Matériau de base prothétique polymérisé selon la revendication 11, caractérisé en comprenant une teneur en monomères résiduels du méthacrylate de méthyle d'inférieure ou égal à 3 % en poids déterminée conformément au norme ISO 20795-1:2013, en particulière il comprend une teneur en monomères résiduels du méthacrylate de méthyle d'inférieure ou égale à 2,5 % en poids, de préférence d'inférieure ou égale à 2,4, 2,3, 2,2, 2,1, 2,05 % en poids, en particulière ayant une plage de variation de +/- 0,05 % en poids.

13. Matériau de base prothétique polymérisé selon la revendication 11 ou 12, **caractérisé en ce que** le matériau de base prothétique a une transparence de supérieure ou égale à 95 &% (mesurée pour des échantillons de couleur de l'épaisseur 3 mm produits dans des moules en métal).

14. Utilisation du matériau de base prothétique selon l'une des revendications précédentes dans le domaine dentaire humain pour produire des prothèses dentaires, des parties des prothèse, pour la fabrication des gouttières occlusales, des guides chirurgicaux pour l'implantology, des protège-dents, pour produire un ébauche fraisage, une prothèse articulaire, une couronne, une prothèse télescopique ou une couronne télescopique, une facette, un bridge dentaire, une dent prothétique, une partie d'implant, un pilier, une superstructure, des appareils et instruments orthodontiques, des corps moulés prothétiques sous forme des os ou des parties de ceci, des corps moulés prothétiques dans le domaine vétérinaire, pour produire une partie de sabot ou des matériaux de réparation de sabots, pour produire du cément d'os, pour produire du cément d'os pour cémenter des prothèse articulaires artificielles, des couronnes, des prothèses télescopiques ou des couronnes télescopiques, des facettes, des bridges dentaires, des dents prothétiques, des implants, des parties d'implant, des piliers, des superstructures.

15. Kit comprenant un matériaux de base prothétique autopolymérisable, le kit comprenant des composantes (A) et (B) séparés, **caractérisé en ce que** le composant (A) comprend
(i) 60 à 85 % en poids methacrylate de méthyle,
(ii) 5 à 20 % en poids au moins un acryloyloxy carbamate N-alkyl- ou N-alkényle-substitué ayant une masse moléculaire d'inférieure ou égale à 250 g/mol,
(iii) 0,5 à 10 % en poids au moins un (méth)acrylate d'uréthane au moins di-fonctionnel,
(iv) 0,05 à 10 % en poids au moins une monomère di-, tri-, tétra- ou multi-fonctionnel autre que le (méth)acrylate d'uréthane,
(v) 0,1 à 10 % en poids des particules polymériques, étant présentes en tant que des particules cœur-coque modifiées par une phase élastique, ayant une taille de particules primaire d'inférieure à 800 nm,
(vi) 0,05 à 2 % au moins un initiateur ou au moins un composant d'un système initiateur pour l'autopolymérisation, les informations précédentes en % en poids étant basées sur la composition totale du composant (A), ainsi que
le composant (B) comprend
(i) 90 à 99,95 % en poids au moins un composant pulvérulent des particules polymériques comprenant au moins trois fractions des taille de particules des particules polymériques, les trois fractions étant sélectionnées parmi des particules polymériques d'une taille de particules moyenne
a) de 25 à inférieure à 40 µm, étant présentes dans 50 à 90 % en poids,
b) de 40 à inférieure à 55 µm, étant présentes dans 0,1 à 20 % en poids, et
c) de 55 à 100 µm, étant présentes dans 0,5 à 30 % en poids, et
(ii) 0,05 à 10 % en poids au moins un initiateur ou au moins un composant d'un système d'initiateur pour l'autopolymérisation, les informations précédentes en % en poids étant basées sur la composition totale du composant (B).
